# EUROPEAN PATENT APPLICATION

(11) **EP 4 834 376 A1**
(43) Date of publication of application: **04.11.2026**
(21) Application number: 26163849.8
(22) Date of filing: 11.03.2026
(51) Int. Cl.: A61K 51/04, A61P 35/00

(54) **PSMA-TARGETING LIGAND COMPOUND, AND CHELATE AND USE THEREOF**

(30) Priority: 28.04.2025 CN 202510549750; 16.09.2025 CN 202511322279
(71) Applicant: Mednovo Group Co. Ltd, Suzhou, Jiangsu 215163 (CN)
(72) Inventor: LI, Peishang, Jiangsu, 215163 (CN); KONG, Xiangsheng, Jiangsu, 215163 (CN); CAI, Chenchen, Jiangsu, 215163 (CN); WANG, Zhiqian, Jiangsu, 215163 (CN); QU, Chunmiao, Jiangsu, 215163 (CN); ZHANG, Mingjie, Jiangsu, 215163 (CN)
(74) Representative: Plougmann Vingtoft a/s

(57) **Abstract**

A prostate-specific membrane antigen (PSMA)-targeting ligand compound and a chelate thereof and use thereof are provided. The PSMA-targeting ligand compound has a structure as shown in formula I, 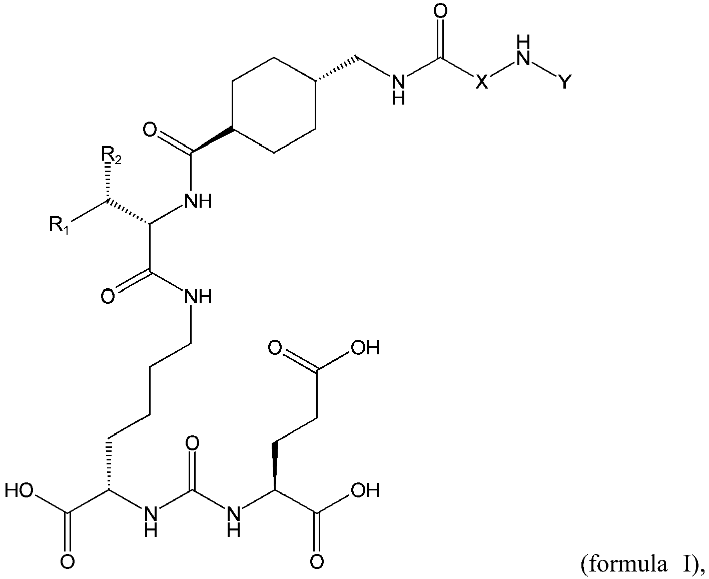 where R₁ is selected from the group consisting of optionally substituted aryl and optionally substituted heteroaryl; R₂ is selected from the group consisting of hydrogen, deuterium, carboxyl, halogen, unsubstituted linear or branched C1-C5 alkyl, linear or branched C1-C5 alkyl substituted with halogen, linear or branched C1-C5 alkylacyl, linear or branched C1-C5 alkyl ester group, hydroxyl, linear or branched C1-C5 alkylthio, and cyano; X is -Z-(CH₂)ₙ-(CR₃R₄)-, where Z is a chemical bond, -NH- or -(CH₂)-, n is 0-5, and R₃ and R₄ are each independently selected from the group consisting of hydrogen, carboxyl, and optionally substituted linear or branched C1-C5 alkyl; and Y is a radioactive metal ion chelating group.

## Description

### TECHNICAL FIELD

The present disclosure belongs to the technical field of pharmaceuticals. In particular, the present disclosure relates to a PSMA-targeting ligand compound, and a chelate thereof and use thereof.

### BACKGROUND

Prostate cancer is a non-negligible disease in the male genitourinary system and one of the most common malignant tumors. According to statistical data, there are about 1,414,259 new cases of prostate cancer, accounting for 7.3% of all malignant tumors, while the disease results in 375,304 deaths, constituting 3.8% of total malignant tumor mortality. Such data highlights that prostate cancer poses substantial threats to men's health, making combating this disease a crucial mission in the medical field.

Prostate-specific membrane antigen (PSMA), a transmembrane glycoprotein, is over-expressed in most prostate cancer cells and metastases thereof. This characteristic makes it become as a critical therapeutic target in metastatic prostate cancer (mPCa). PSMA-617, also known as vipivotide tetraxetan, is a potent PSMA inhibitor with an inhibition constant (Ki) as low as 0.37 nM. It is constituted of a targeting group, namely glutamate-urea-lysine, DOTA capable of chelating ⁶⁸Ga or ¹⁷⁷Lu, and a linker connecting these two components. Glutamate-urea-lysine can selectively bind to PSMA. PSMA-617, when conjugated with the radioactive isotope Lutetium-177 (¹⁷⁷Lu), forms a radionuclide drug conjugate (RDC) ¹⁷⁷Lu-PSMA-617. The medicament has been approved for marketing by the FDA under the trade name Pluvicto, and is used for the treatment of PSMA-positive metastatic castration-resistant prostate cancer (mCRPC) in patients. It has been revealed in the clinical phase III VISION trial that ¹⁷⁷Lu-PSMA-617 significantly prolonged the survival in patients with metastatic castration-resistant prostate cancer. Furthermore, PSMA-617 is also used in PSMA PET/CT imaging for the assessment of PSMA expression, enhancing the sensitivity and accuracy of prostate cancer diagnosis.

PSMA-617 has achieved success in the treatment and diagnosis of prostate cancer; nevertheless, clinical applications have revealed its deficiencies in pharmacokinetic profile. Suboptimal parameters in terms of drug absorption, distribution, and metabolism limit the therapeutic efficacy of the radionuclide. Consequently, there remains a pressing need in the pharmaceutical field for the optimization and improvement of radionuclide-conjugated ligand targeting a prostate-specific membrane antigen to meet the higher demands of clinical therapy.

### SUMMARY

In view of the above problems, an object of the present disclosure is to provide a PSMA-targeting ligand compound, a chelate thereof and use thereof. Through deep researches, the inventors have discovered that a new class of PSMA-targeting ligand compounds with markedly improved pharmacokinetic profiles can be obtained by modifying the chemical structure of PSMA-617 and screening for related biological activity. These compounds exhibit more efficient absorption and good stability in the human body, and are also effectively taken up and internalized by cancerous cells. Therefore, conjugating the ligand compound with a radionuclide can yield a significantly improved anti-cancer drug or a diagnostic agent with better imaging performance.

The above-mentioned object of the present disclosure is achieved by the following technical solutions provided herein:

In the first aspect, the present disclosure provides a PSMA-targeting ligand compound having a structure as shown in formula I, or a stereoisomer, a tautomer, a hydrate, a solvate, a pharmaceutically acceptable salt or ester thereof, 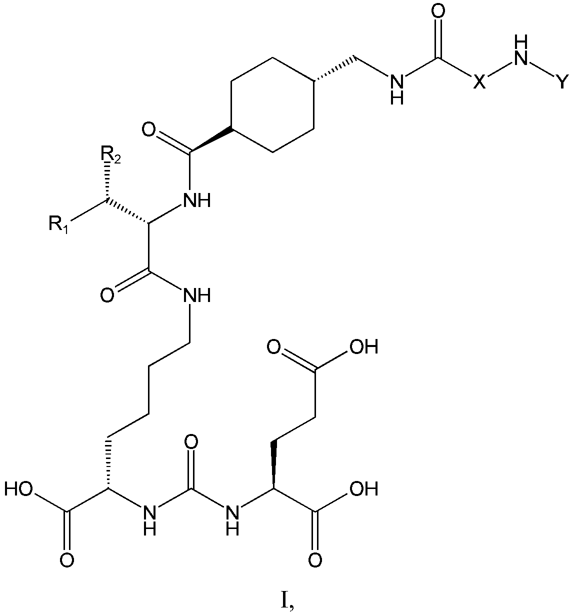 wherein R₁ is selected from the group consisting of optionally substituted aryl and optionally substituted heteroaryl, a substituent in the optionally substituted aryl or the optionally substituted heteroaryl is selected from the group consisting of: deuterium, carboxyl, halogen, linear or branched C1-C5 alkyl substituted with halogen, linear or branched C1-C5 alkylacyl, linear or branched C1-C5 alkyl ester group, hydroxyl, linear or branched C1-C5 alkylthio, cyano, linear or branched C1-C5 alkoxy, amino, nitro, phenylsulfonamido, linear or branched C1-C5 alkylamido, linear or branched C2-C6 alkenyl, and linear or branched C2-C6 alkynyl; R₂ is selected from the group consisting of: hydrogen, deuterium, carboxyl, halogen, unsubstituted linear or branched C1-C5 alkyl, linear or branched C1-C5 alkyl substituted with halogen, linear or branched C1-C5 alkylacyl, linear or branched C1-C5 alkyl ester group, hydroxyl, linear or branched C1-C5 alkylthio, cyano, linear or branched C1-C5 alkoxy, amino, nitro, phenylsulfonamido, linear or branched C1-C5 alkylamido, linear or branched C2-C6 alkenyl and linear or branched C2-C6 alkynyl; X is -Z-(CH₂)ₙ-(CR₃R₄)-, wherein Z is a chemical bond, -NH- or -(CH₂)-, n is 0-5, R₃ and R₄ are each independently selected from the group consisting of: hydrogen, carboxyl and optionally substituted linear or branched C1-C5 alkyl, and a substituent in the optionally substituted linear or branched C1-C5 alkyl is selected from the group consisting of: halogen, hydroxyl, amino, carboxyl, amido, -CN, -CF₃, nitro, sulfonyl, linear or branched C1-C5 alkoxy, linear or branched C1-C5 alkylamino, linear or branched C2-C6 alkenyl, linear or branched C2-C6 alkynyl, five-membered or six-membered aryl, linear or branched C1-C5 heteroalkyl, five-membered or six-membered heteroaryl, five-membered or six-membered heterocycloalkyl, linear or branched C1-C5 alkyl substituted with five-membered or six-membered aryl, five-membered or six-membered aryl substituted with linear or branched C1-C5 alkyl, five-membered or six-membered heteroaryl substituted with linear or branched C1-C5 alkyl, linear or branched C1-C5 alkyl substituted with five-membered or six-membered heteroaryl, and biphenyl; and Y is a radioactive metal ion chelating group.

According to some embodiments of the present disclosure, under a condition that Z is - NH-, R₃ and R₄ are both hydrogen; under a condition that Z is -(CH₂)-, one of R₃ and R₄ is hydrogen, and the other is carboxyl; or under a condition that Z is a chemical bond, n = 0, and one of R₃ and R₄ is hydrogen, the other is linear or branched C1-C5 alkyl substituted with carboxyl.

According to some embodiments of the present disclosure, R₁ is selected from optionally substituted phenyl and optionally substituted naphthyl, wherein a substituent in the optionally substituted phenyl or the optionally substituted naphthyl is selected from the group consisting of halogen, such as F-, Cl-, Br- or I-, and cyano, trifluoromethyl, difluoromethyl and monofluoromethyl.

According to some embodiments of the present disclosure, R₂ is selected from the group consisting of hydrogen and unsubstituted linear or branched C1-C5 alkyl.

According to some embodiments of the present disclosure, X is -NH-(CH₂)-, -(CH₂)-(CH₂)-(CHCOOH)-, -(CH-CH₂-CH₂COOH)-, -(CH-CH₂-COOH)-, -NH-(CH₂)-(CH₂)-, -(CH₂)-(CHCOOH)- or -(CH₂)-(CH₂)-(CH₂)-(CHCOOH)-.

According to some embodiments of the present disclosure, Y is the radioactive metal ion chelating group formed from a chelating agent as follows: 1,4,7,10-tetraazacyclododecane-1,4,7,10-tetraacetic acid (DOTA), 1,4,7-triazacyclononane- 1,4,7-triacetic acid (NOTA), 1,4,7-triazacyclononane triacetic acid modified with an amino acid (NOTA-AA), diethylenetriamine pentaacetic acid (DTPA), 1,4,7-triazacyclononane-1,4-diacetic acid (NODA), 2-(4,7-bis(carboxymethyl)-1,4,7-triazonon-1-yl)glutaric acid (NODAGA), 1,4,7,10-tetraazacyclododecane-1,4,7,10-tetramethylene phosphonic acid (DOTP), 1,4,8,11-tetraazacyclotetradecane-1,4,8,11-tetraacetic acid (TETA), diacetylbis(N4- methyl-3-thiosemicarbazone) (ATSM), pyruvaldehyde bis(N4,N4-dimethylthiosemicarbazone) (PTSM), ethylenediamine tetraacetic acid (EDTA), trisodium ethylenediamine disuccinate (EC), N,N'-bis(2-hydroxybenzyl)-ethylenediamine-N,N-diacetic acid (HBED), N,N'-bis(2-hydroxybenzyl)ethylenediamine-N,N'-diacetic acid ketone (HBEDCC), monobenzylamino diacetic acid (SBAD), N,N'-bis(3-aminopropyl)ethylenediamine (BAPEN), desferal (Df), N'-{5-[acetyl(hydroxyl)amino]pentyl}-N-[5-({4-[5-aminopentyl)(hydroxyl)amino]-4-oxobutanoyl}amino)pentyl]-N-hydroxylsuccinamide (DFO), 1,4,7-triazacyclononane (TACN), 1,4,7- triazacyclononane-1,7-diacetic acid/1,4,7-triazacyclononane-1,4,7-triacetic acid maleimide (NO2A/NOTAM), 1,4,7,10-tetraazacyclododecane-1,7-diacetic acid (CB-DO2A), 1,4,7,10-tetraazacyclododecane (Cyclen), sodium 2,2',2"-(1,4,7,10-tetraazacyclododecane-1,4,7-triyl)triacetate (DO3A), 1,4,7,10-tetraazacyclododecane-1,4,7-triacetic acid-10-methylphosphonic acid (DO3AP), 6-hydrazinonicotinic acid N-hydroxysuccinimide ester hydrochloride (HYNIC), S-acetylmercaptoacetyltriserine (MAS3), mercaptoacetyltriglycine (MAG3), isonitrile, and derivatives thereof.

According to some embodiments of the present disclosure, R₁ is selected from the group consisting of the following groups: 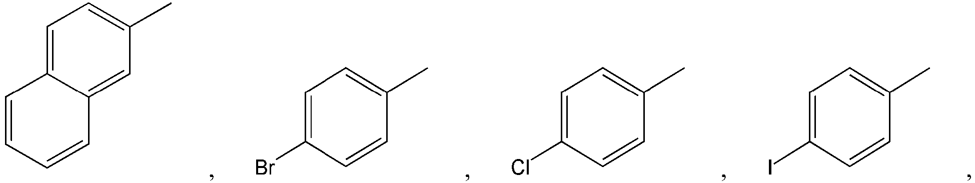 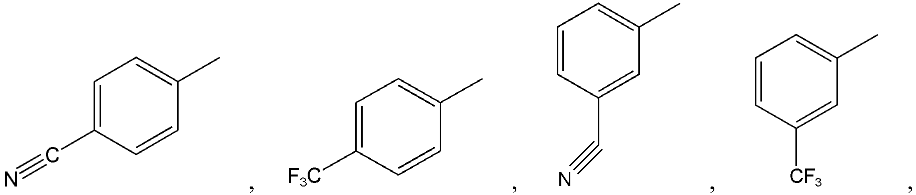 and

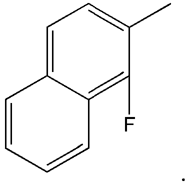

According to some embodiments of the present disclosure, Y is

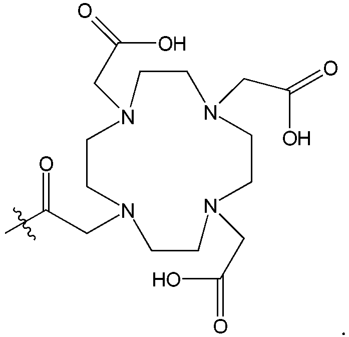

According to some specific embodiments of the present disclosure, the PSMA-targeting ligand compound or the stereoisomer thereof is selected from the group consisting of the following compounds:

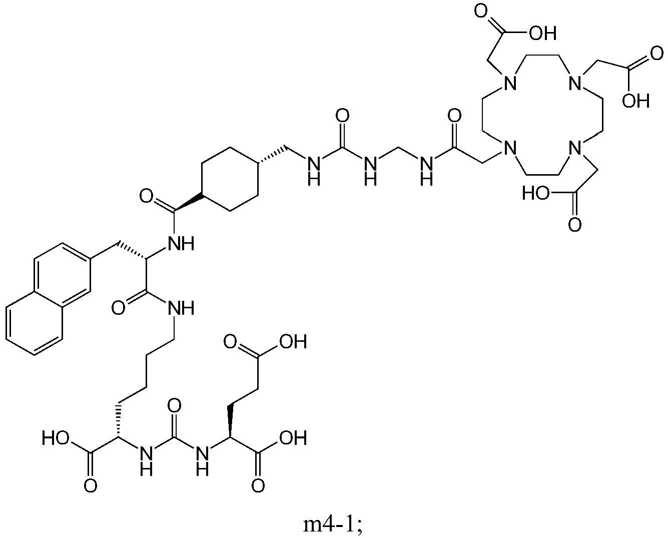

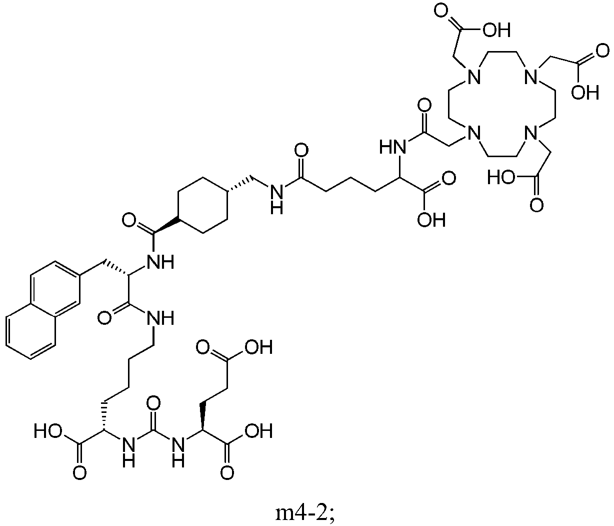

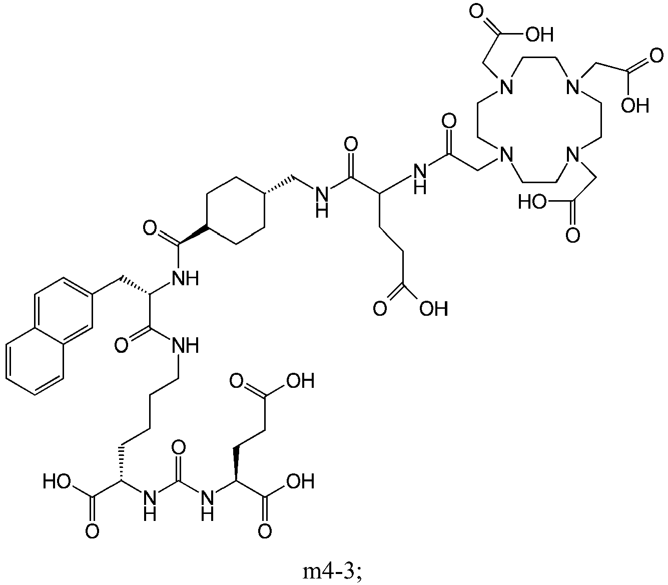

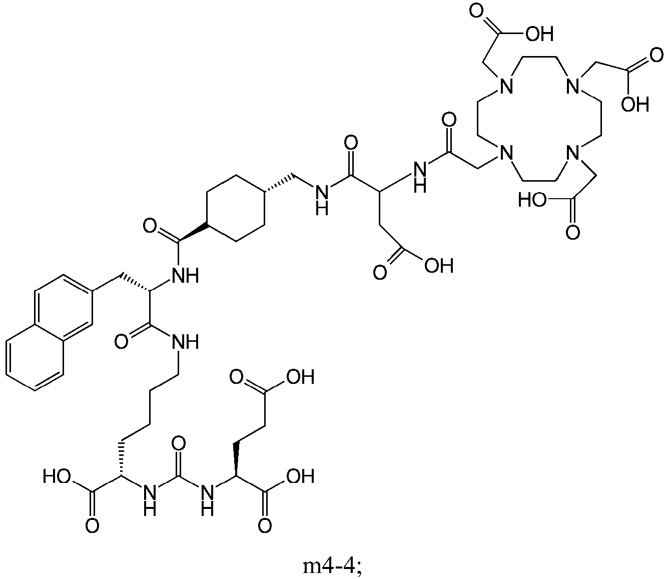

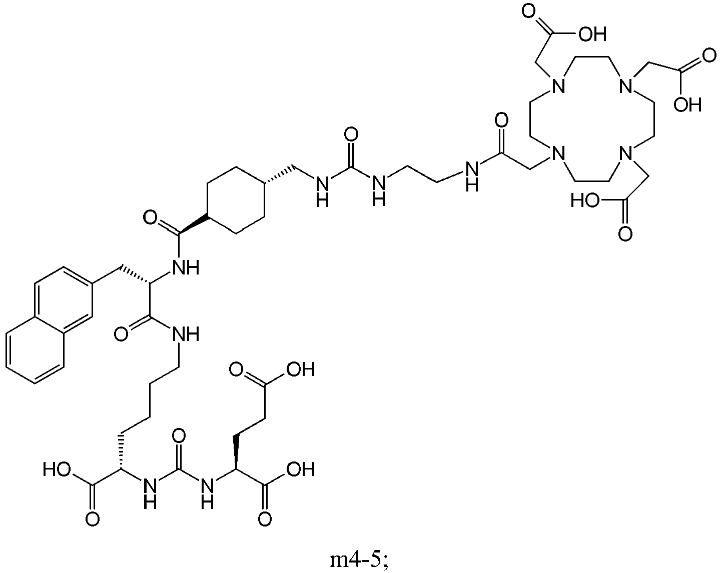

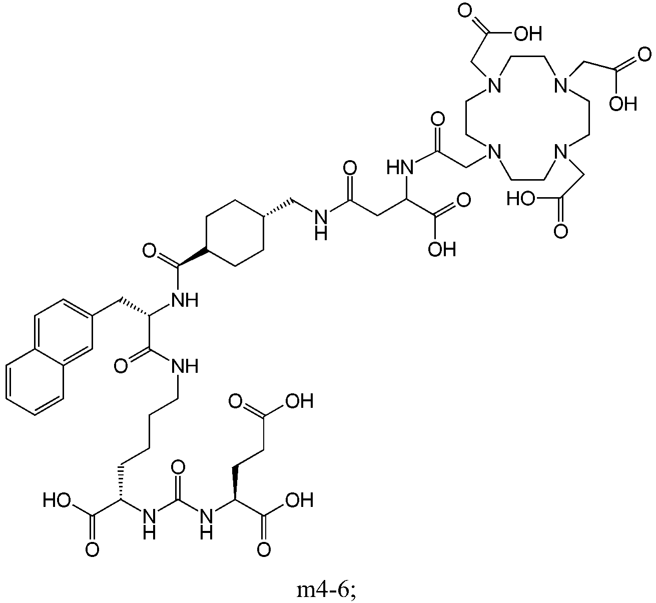

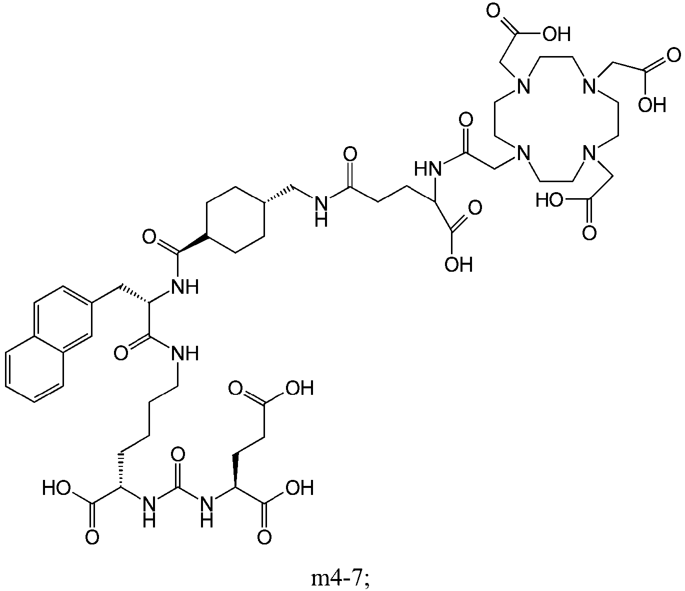

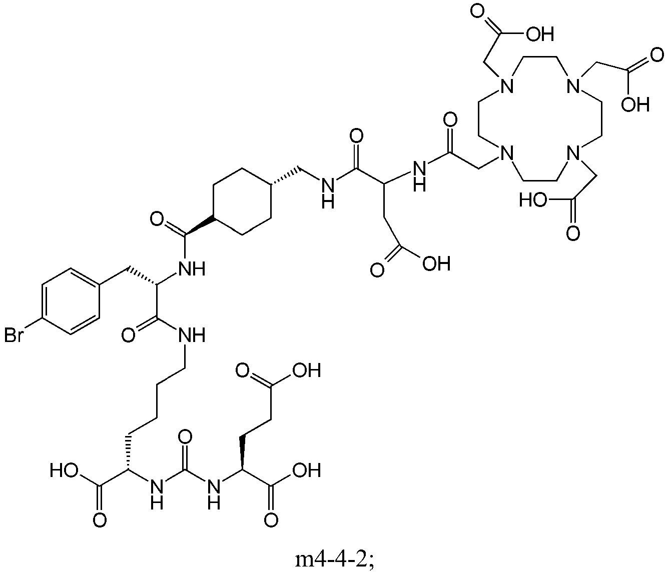

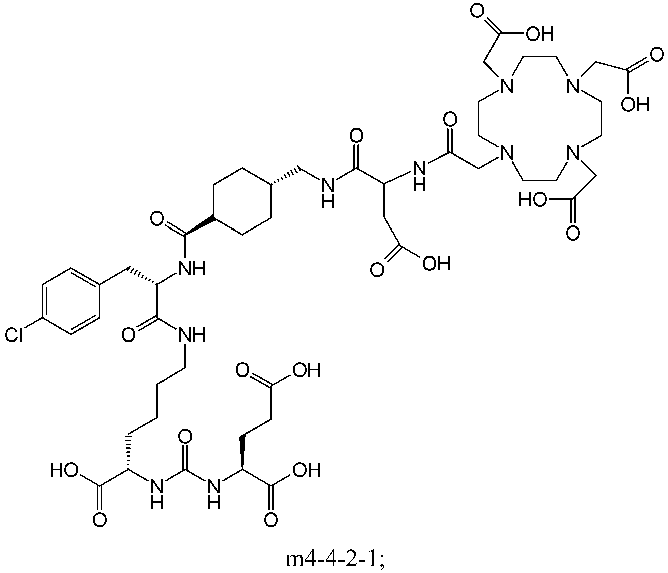

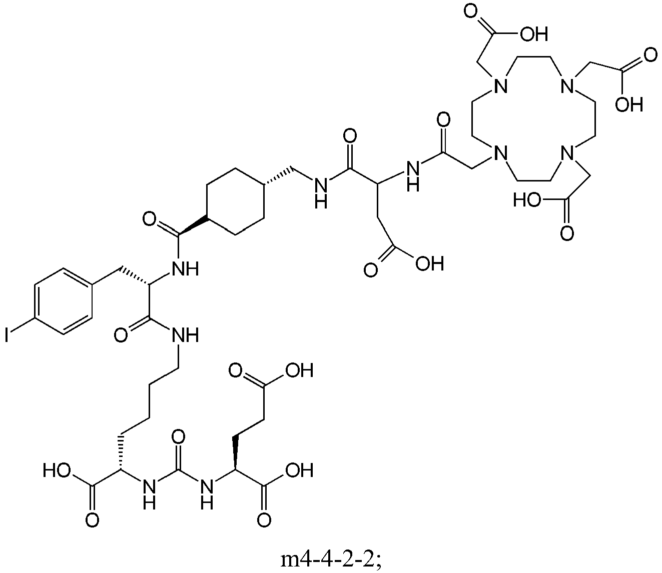

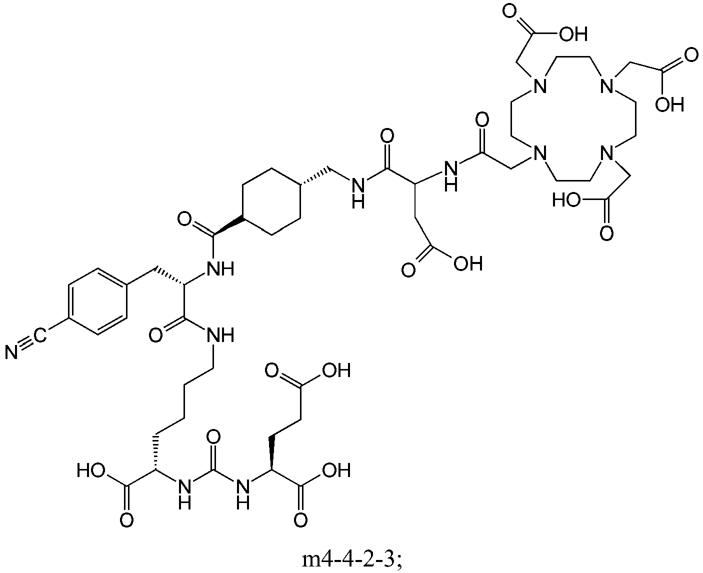

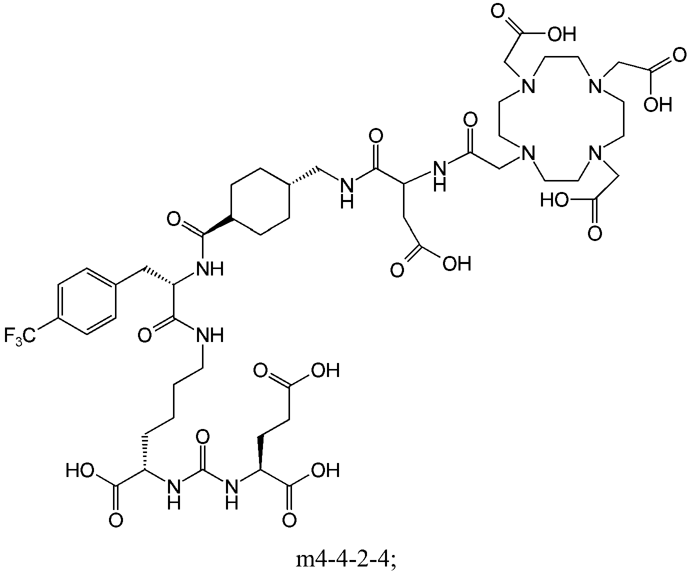

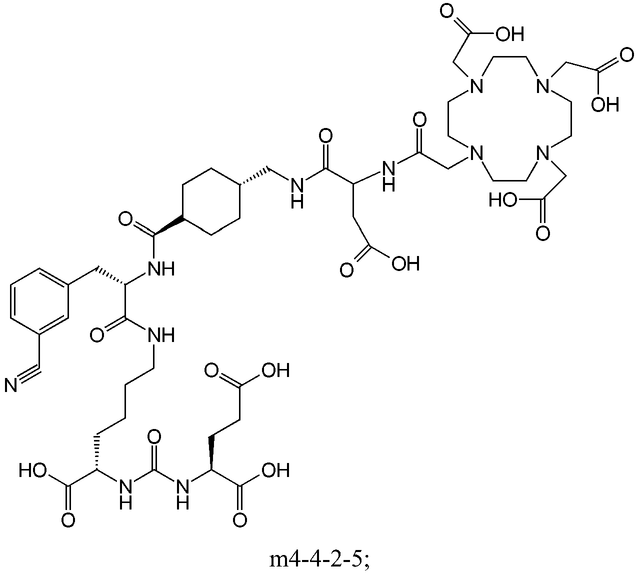

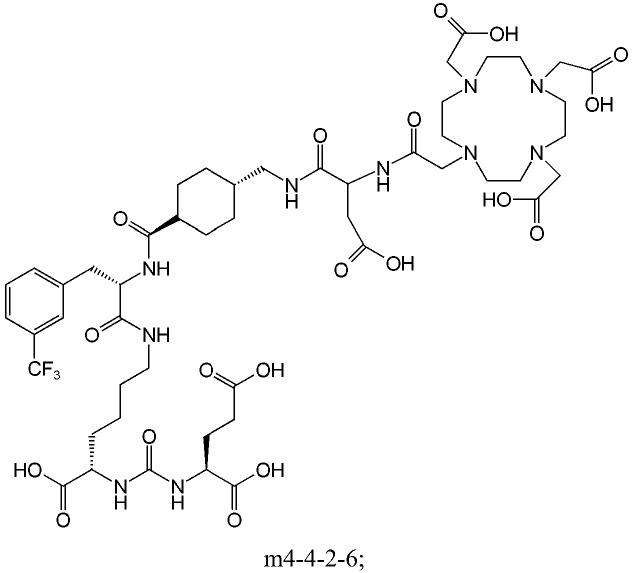

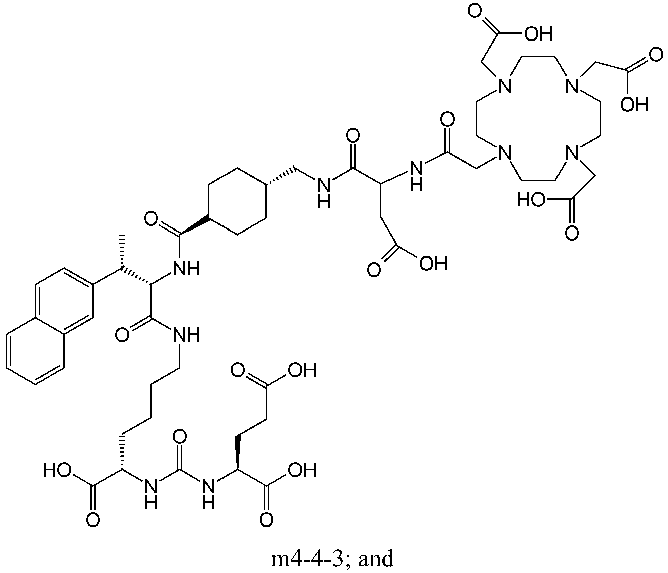

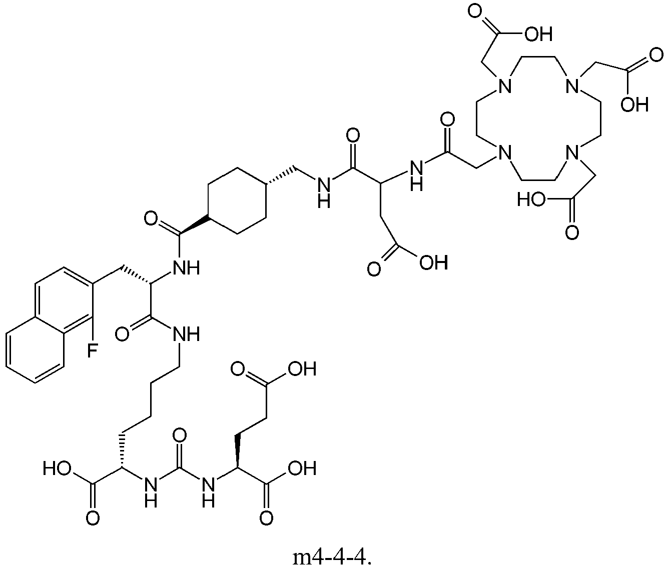

In a second aspect, the present disclosure provides a method for preparing the PSMA-targeting ligand compound or the stereoisomer, the tautomer, the hydrate, the solvate, the pharmaceutically acceptable salt or ester thereof according to the first aspect of the present disclosure, the method including the following steps:
(1) reacting a compound as shown in formula II with a compound as shown in formula III in an organic solvent containing a condensing reagent and an organic amine to produce a compound as shown in formula IV; 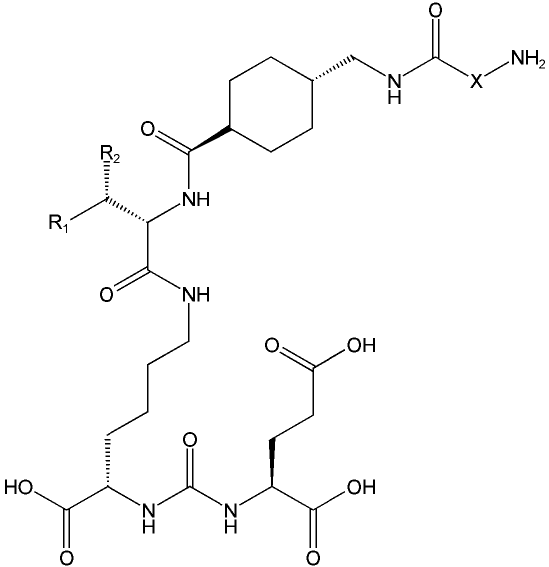 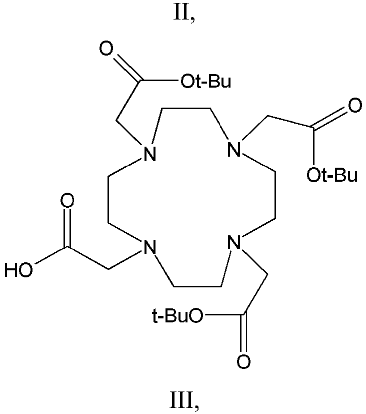 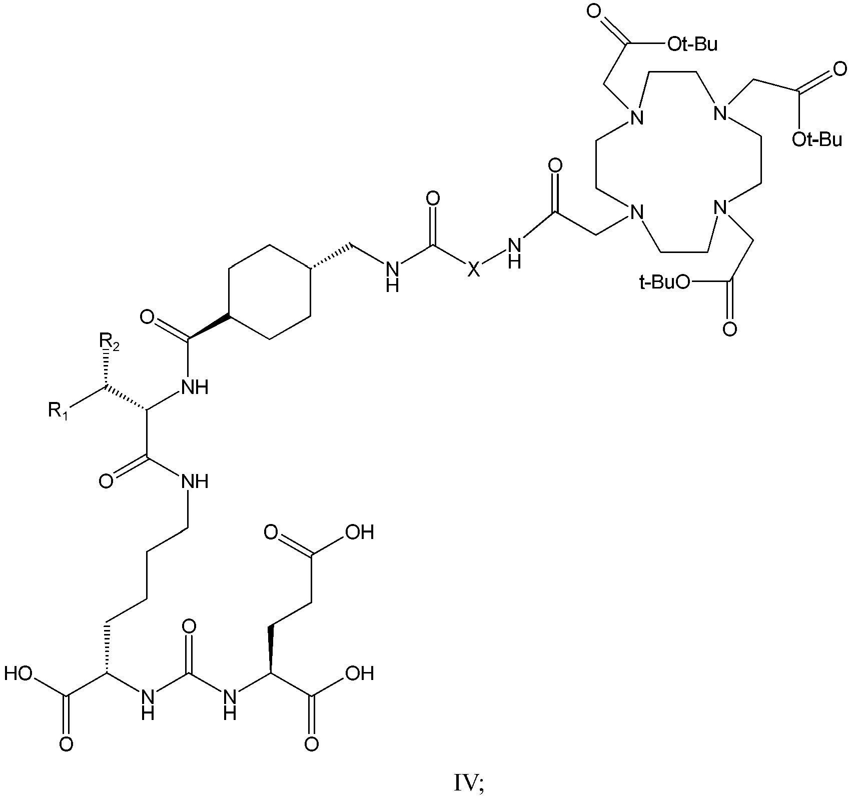 and
(2) reacting the compound as shown in formula IV in an organic solvent containing a deprotection reagent to produce the PSMA-targeting ligand compound as shown in formula I.

According to some embodiments of the present disclosure, the condensing reagent is at least one selected from the group consisting of 2-(7-azobenzotriazole)-N,N,N',N'-tetramethyluronium hexafluorophosphate (HATU), N,N'-dicyclohexylcarbodiimide (DCC), 1-(3-dimethylaminopropyl)-3-ethylcarbodiimide (EDCI), (benzotriazol-1-yloxy)tripyrrolidnophosphonium hexafluorophosphate (ByBOP), and 1-propylphosphonic cyclic anhydride (T3P).

According to some embodiments of the present disclosure, the organic amine is one of N,N-diisopropylethylamine and triethylamine, or a mixture of the N,N-diisopropylethylamine (DIEA) and the triethylamine (Et₃N).

According to some embodiments of the present disclosure, the deprotection reagent is at least one selected from the group consisting of trifluoroacetic acid (TFA), a solution of hydrogen chloride in dioxane, a solution of hydrogen chloride in methanol, and a solution of hydrogen chloride in ethyl acetate.

According to some embodiments of the present disclosure, the organic solvent is at least one selected from the group consisting of N,N-dimethylformamide (DMF), tetrahydrofuran (THF), and dichloromethane (DCM).

In a third aspect, the present disclosure provides a chelate including the PSMA-targeting ligand compound or the stereoisomer, the tautomer, the hydrate, the solvate, the pharmaceutically acceptable salt or ester thereof according to the first aspect of the present disclosure, and a radioactive metal ion chelated thereto.

According to some embodiments of the present disclosure, a radioactive metal is one or more selected from the group consisting of ¹⁷⁷Lu, ¹²⁵I, ¹³¹I, ²¹¹At, ¹¹¹In, ¹⁵³Sm, ¹⁸⁶Re, ¹⁸⁸Re, ⁶⁷Cu, ²¹²Pb, ²²⁵Ac, ²¹³Bi, ²¹²Bi, ²¹²Pb, and ⁶⁸Ga.

According to some embodiments of the present disclosure, the radioactive metal is ¹⁷⁷Lu.

In a fourth aspect, the present disclosure provides a pharmaceutical composition for use in the treatment of a tumor, the pharmaceutical composition including the chelate according to the third aspect of the present disclosure, and optionally, at least one pharmaceutically acceptable carrier.

In a fifth aspect, the present disclosure provides a reagent composition for use in the diagnosis of a tumor, the reagent composition including the chelate according to the third aspect of the present disclosure, and optionally, at least one diagnostically acceptable carrier.

In a sixth aspect, the present disclosure provides use of the PSMA-targeting ligand compound or the stereoisomer, the tautomer, the hydrate, the solvate, the pharmaceutically acceptable salt or ester thereof according to the first aspect of the present disclosure in preparation of a PSMA inhibitor.

Correspondingly, the present disclosure provides a method for inhibiting PSMA, including: administering an effective amount of the PSMA-targeting ligand compound or the stereoisomer, the tautomer, the hydrate, the solvate, the pharmaceutically acceptable salt or ester thereof according to the first aspect of the present disclosure to a subject.

In a seventh aspect, the present disclosure provides use of the chelate according to the third aspect of the present disclosure in preparation of a medicament for the treatment of a tumor.

Correspondingly, the present disclosure provides a method for treating a tumor, including: administering a therapeutically effective amount of the chelate according to the third aspect of the present disclosure to a subject.

In the eighth aspect, the present disclosure provides use of the chelate according to the third aspect of the present disclosure in preparation of a reagent for the diagnosis of a tumor.

According to some embodiments of the present disclosure, the reagent for the diagnosis of a tumor is a tumor imaging agent.

Correspondingly, the present disclosure provides a method for imaging a tumor, including: administering an effective amount of the chelate according to the third aspect of the present disclosure to a subject, and performing imaging.

According to some embodiments of the present disclosure, the tumor is at least one selected from the group consisting of prostate cancer, renal carcinoma, and gastric cancer.

According to some embodiments of the present disclosure, the tumor is prostate cancer. In particular, the prostate cancer is at least one selected from the group consisting of castration-resistant prostate cancer, metastatic castration-resistant prostate cancer, and PSMA-positive prostate cancer.

As used herein, the term "subject" means a human or animal. Generally, the animal is a vertebrate such as a primate, rodent, domestic animal, or game animal. Primates include chimpanzees, cynomolgus monkeys, spider monkeys, and macaques, e.g., Rhesus monkey. Rodents include mice, rats, marmots, ferrets, rabbits and hamsters. Domestic and game animals include cows, horses, pigs, deer, bison, buffalo, felines, e.g., domestic cat, and canines, e.g., dog, fox, wolf. In some embodiments, the subject is a mammal. The mammal can be a human, non-human primate, mouse, rat, dog, cat, horse, or cow, but are not limited to these examples. In addition, the methods described herein can be used to treat domesticated animals and/or pets. The term does not denote a particular age or gender. Thus, adult and newborn subjects, as well as fetuses, whether male or female, are intended to be included within the scope of this term.

As used herein, the term "administer" involves any route for delivering the active substance, i.e., the PSMA-targeting ligand compound or the stereoisomer, the tautomer, the hydrate, the solvate, the pharmaceutically acceptable salt or ester, chelate or composition thereof to a subject. In some embodiments, the active substance described herein is administered enterally to the small intestine. Routes of delivery may include non-invasive oral (through the mouth), topical (skin), transmucosal (nasal, buccal/sublingual, vaginal, ocular and rectal) and inhalation routes, as well as parenteral routes, and other methods known in the art. Parenteral administration refers to a route of delivery that is generally associated with injection, including intraorbital, infusion, intraarterial, intracarotid, intracapsular, intracardiac, intradermal, intramuscular, intraperitoneal, intrapulmonary, intraspinal, intrasternal, intrathecal, intrauterine, intravenous, subarachnoid, subcapsular, subcutaneous, transmucosal, or transtracheal routes. When administered via the parenteral route, the composition may be in the form of a solution or a suspension for infusion or for injection, or as lyophilized powders.

As used herein, the term "effective amount" refers to the amount of the active substance to decrease at least one or more symptoms of a disease or disorder, and relates to a sufficient amount of the active substance to provide the desired effect. The phrase "therapeutically effective amount" as used herein means a sufficient amount of the active substance to treat a disorder, at a reasonable benefit/risk ratio applicable to any medical treatment.

As used herein, the term "treatment" refers to therapeutic treatment, wherein the purpose thereof is to reverse, alleviate, ameliorate, inhibit, slow down or stop the progression or severity of a condition associated with, a disease or disorder. The term "treating" includes reducing or alleviating at least one adverse effect or symptom of a condition, disease or disorder. Treatment is generally "effective" if one or more symptoms or clinical markers are reduced. Alternatively, treatment is "effective" if the progression of a disease is reduced or halted. That is to say, "treatment" includes not just the improvement of symptoms or markers, but also a cessation or at least slowing of the progression or worsening of symptoms that would be expected in absence of the treatment. Beneficial or desired clinical results include, but are not limited to, alleviation of one or more symptom(s), reduction in the extent of the disease, stabilized (i.e., not worsening) state of disease, delay or slowing of disease progression, amelioration or alleviation of the disease state, and remission (whether partial or total), whether detectable or undetectable. The term "treatment" of a disease also includes achieving relief from the symptoms or adverse effect of the disease (including palliative treatment).

A therapeutically or prophylactically significant reduction in a symptom is defined as, e.g. at least about 10%, at least about 20%, at least about 30%, at least about 40%, at least about 50%, at least about 60%, at least about 70%, at least about 80%, at least about 90%, at least about 100%, at least about 125%, at least about 150% or more in a measured parameter as compared with the status of a control or non-treated subject prior to the administration of the active substance described herein. Measured or measurable parameters include clinically detectable markers of disease, for example, elevated or depressed levels of a biological marker, as well as parameters related to a clinically accepted scale of symptoms or markers for the diseases. It will be understood, however, that the total daily administration amount of the active substance disclosed herein is decided by the attending physician within the scope of sound medical judgment. The exact administration amount required varies depending on factors such as the type of disease being treated, and gender, age, and weight of the subject.

As used herein, the term "optionally" may be taken to mean that the subsequently described structure, event or circumstance may or may not occur, and that the description includes both instances where the event occurs and instances where it does not occur.

Embodiments of the present disclosure have at least the following beneficial effects:
1. The present disclosure employs an innovative approach of molecular structure optimization to ingeniously fuse a large-molecular-weight radionuclide chelating group with a PSMA-targeting group, successfully obtaining a PSMA-targeting ligand compound and a stereoisomer, a tautomer, a hydrate, a solvate, a pharmaceutically acceptable salt or ester thereof. Experiments show that the compound of the present disclosure exhibits comparable performance in radiodiagnostic applications (PET/CT (positron emission tomography / computed tomography) or SPECT/CT scanning (single photon emission computed tomography/computed tomography) than currently marketed PSMA diagnostic products, showing no significant differences.
2. The compound of the present disclosure exhibits outstanding advantages in clinical diagnostic applications. Following injection of the radiolabeled agent in the patients, PET/CT or SPECT/CT scanning is conducted, imaging results demonstrate a ultra high tumor-to-background ratio (target to background ratio). This enables clinicians to accurately obtain critical diagnostic information, including tumor size, precise location, and malignancy level, from the precise imaging results.
3. From the perspective of molecular structure design, the present disclosure achieves a groundbreaking multi-functional design in a single molecule through innovative optimization strategies. The same molecular structure can be flexibly loaded with either diagnostic radionuclides or therapeutic radionuclides. This enables combined diagnostic and therapeutic effect or even multiple synergistic effects, genuinely advancing the medical technology innovation of "integrated diagnosis and treatment".
4. Through precise molecular structure optimization, the drug molecule of the present disclosure exhibits significantly enhanced tumor cell targeting performance. Drug molecules have a very high affinity for tumor cells, resulting in prolonged retention at the lesion site alongside rapid clearance from the systemic circulation. The drug's prolonged *in vivo* half-life allows for higher drug accumulation in tumor site, and further reduces the dose and frequency of administration, effectively mitigating potential toxicity risks.

### BRIEF DESCRIPTION OF THE DRAWINGS

The embodiments of the present disclosure are described in detail below with reference to the accompanying drawings, in which:
FIG. 1, FIG. 2A and FIG. 3 show the liquid chromatography mass spectrometry (LC-MS), high performance liquid chromatography (HPLC), and hydrogen nuclear magnetic resonance (¹H-NMR) spectrum, respectively, of compound m4-4, and FIG. 2B shows integration results of FIG. 2A;
FIG. 4, FIG. 5A and FIG. 6 show the LC-MS, HPLC, and ¹H-NMR spectrum, respectively, of compound m4-4-2, and FIG. 5B shows integration results of FIG. 5A; and
FIG. 7 to FIG. 8 show the tissue distribution of the radiolabeled compounds ¹⁷⁷Lu-PSMA-617 and ¹⁷⁷Lu-m4-4, respectively.

### DETAILED DESCRIPTION OF THE EMBODIMENTS

The present disclosure is further described in detail below in conjunction with specific examples, and the examples given are only used to illustrate the present disclosure, but are not to limit the scope of the present disclosure.

The chemical reagents used in the Examples were all commercially available.

### Example 1 Synthesis of compound m4-4

### Step 1: Synthesis of compound m4-4-a

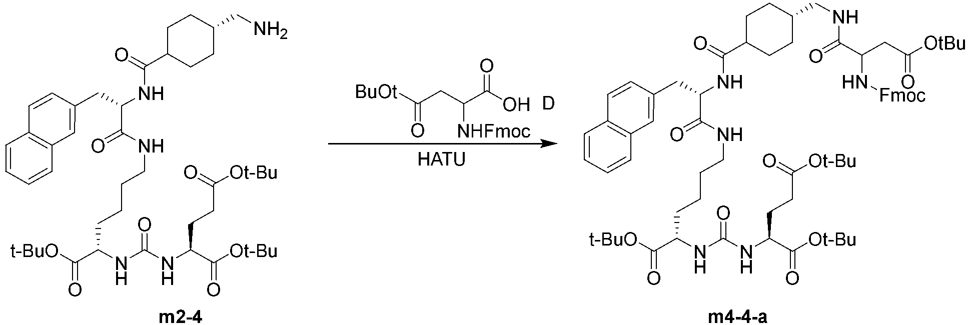

At room temperature, in a 100 mL single-neck flask filled with raw material m2-4 (1.7 g, 2.1 mmol, 1.0 eq.) dissolved in DMF (30 mL), raw material D (1.27 g, 3.1 mmol, 1.5 eq.), DIEA (0.68 mL, 4.1 mmol, 2.0 eq.), and HATU (1.18 g, 3.1 mmol, 1.5 eq.) were separately added, and a resulting mixture was stirred at room temperature overnight; upon product formation as monitored by LCMS, the reaction was stopped. Water and ethyl acetate were added thereto and subjected to extraction and liquid layering. A resulting organic phase was washed with saturated brine, and after separation, a resulting organic phase was concentrated. A resulting crude product was purified by silica gel column chromatography (DCM : MeOH = 5 : 1-2 : 1) to yield product m4-4-a (4 g).

### Step 2: Synthesis of compound m4-4-b

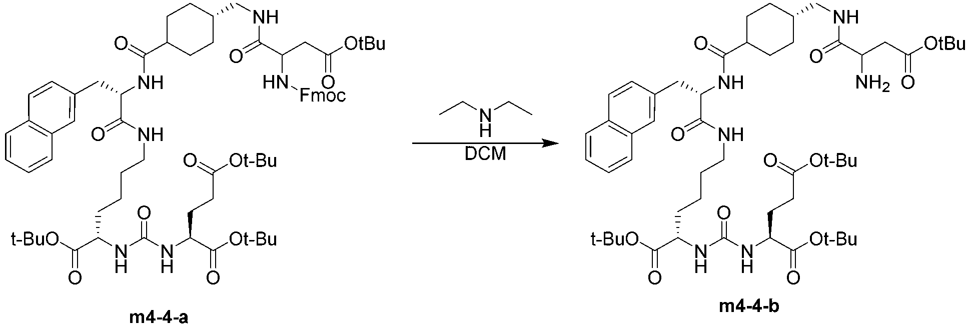

At room temperature, in a 100 mL single-neck flask, raw material m4-4-a (3.0 g, 2.46 mmol, 1.0 eq.) was added, followed by adding DCM (40 mL) thereto, and the m4-4-a was dissolved by stirring. Diethylamine (7.65 mL, 73.9 mmol, 30 eq.) was then added thereto and a resulting mixture was stirred at 25°C for 2 hours. Upon new product spot formation and complete consumption of the raw material as monitored by LCMS, the reaction was stopped. The reaction mixture was concentrated to remove the solvent DCM and diethylamine. Water and ethyl acetate were then added thereto. A resulting mixture was stirred for liquid layering. Resulting organic phases were combined and concentrated to dryness to yield a crude product. The crude product was purified by silica gel column chromatography (DCM : MeOH = 20 : 1-10 : 1) to yield product m4-4-b (2.75 g) as a pale yellow viscous liquid.

### Step 3: Synthesis of compound m4-4-c

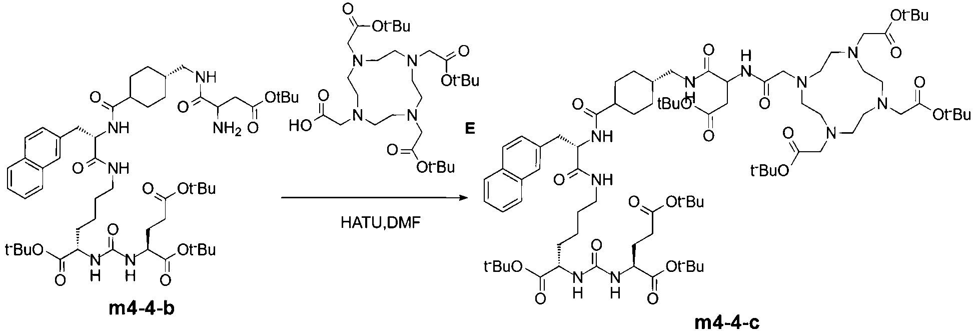

At room temperature, in a 100 mL single-neck flask filled with raw material m4-4-b (2.75 g, 2.76 mmol, 1.0 eq.) dissolved in DMF (40 mL), raw material E (2.37 g, 4.1 mmol, 1.5 eq.), DIEA (0.91 mL, 5.5 mmol, 2.0 eq.) and HATU (1.18 g, 3.1 mmol, 1.5 eq.) were separately added, and a resulting mixture was stirred at room temperature overnight. Upon product formation as monitored by LCMS, the reaction was stopped. Water and ethyl acetate were added and subjected to extraction and liquid layering. A resulting organic phase was washed with saturated brine, and after separation, a resulting organic phase was concentrated. A resulting crude product was purified by silica gel column chromatography (DCM : MeOH = 5 : 1-2 : 1) to yield product m4-4-c, which was then purified by preparative high-performance liquid phase chromatography to yield m4-4-c (1.03 g) as a white solid.

### Step 4: Synthesis of compound m4-4

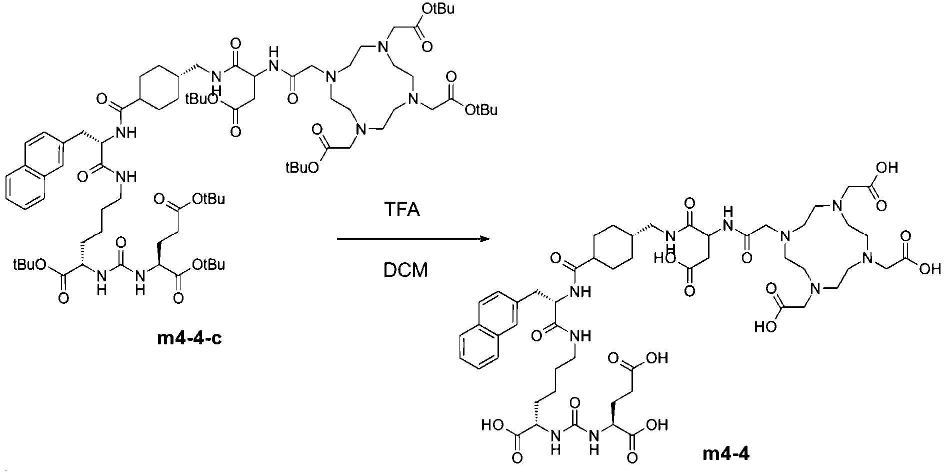

At room temperature, in a 50 mL single-neck flask, raw material m4-4-c (1.03 g, 0.67 mmol) was added, followed by adding DCM (10 mL), and the m4-4-c was dissolved by stirring. TFA (15 mL) was added and a resulting mixture was reacted at 25°C for 2 h. Upon product formation and complete consumption of the raw material as monitored by LCMS, the reaction was stopped. Saturated brine and ethyl acetate were added thereto and a resulting mixture was stirred for liquid layering. A resulting organic phase was collected, concentrated under reduced pressure and then purified by preparative high-performance liquid phase chromatography (Welch Xtimate C18, 21.2 × 250 mm, 5 µm) to yield m4-4 (210 mg, with a yield of 26.2%, and a purity of 95.9%) as a white solid.

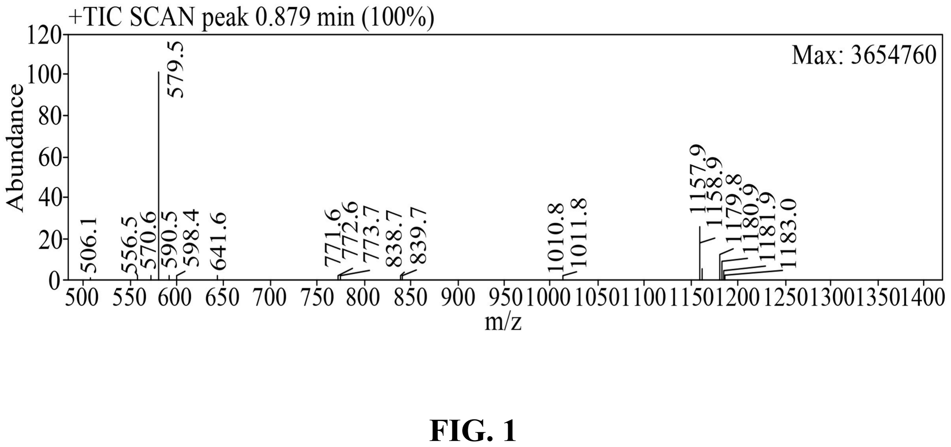

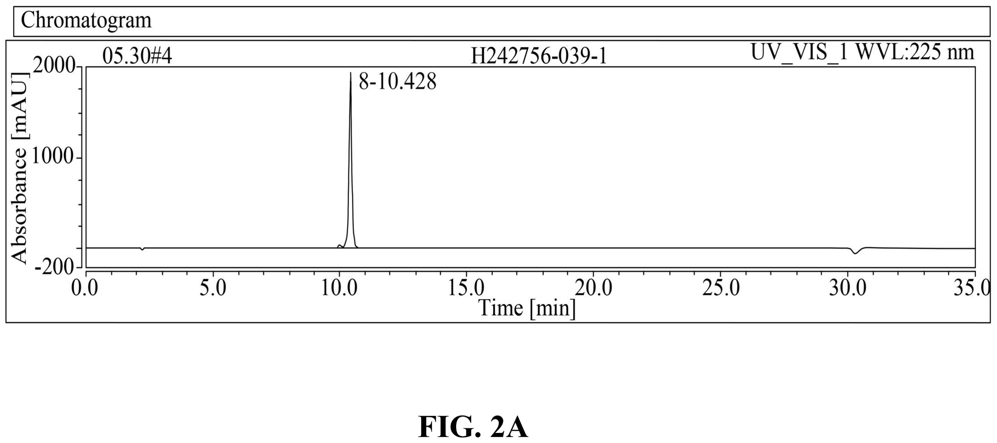

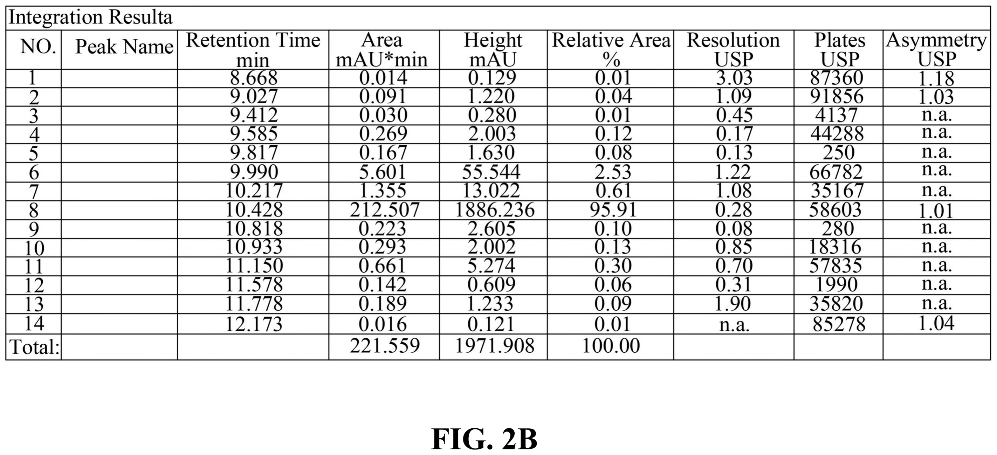

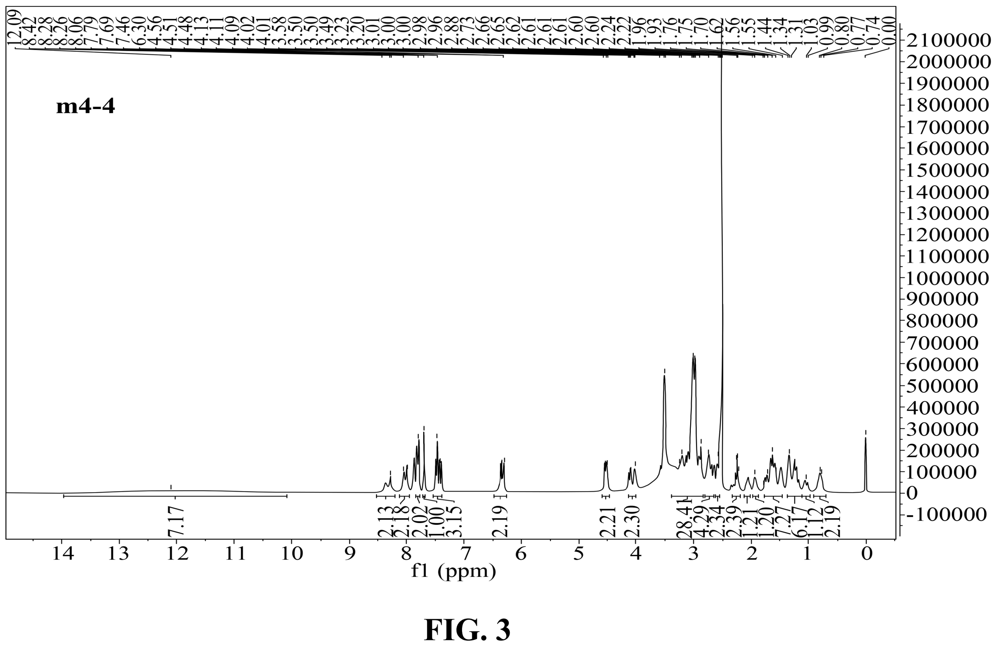

The LC-MS, HPLC and ¹H-NMR spectra of m4-4 are shown in FIG. 1, FIG. 2A and FIG. 3, respectively.

### Example 2 Synthesis of compound m4-4-2

### Step 1: Synthesis of compound m4-4-2-a

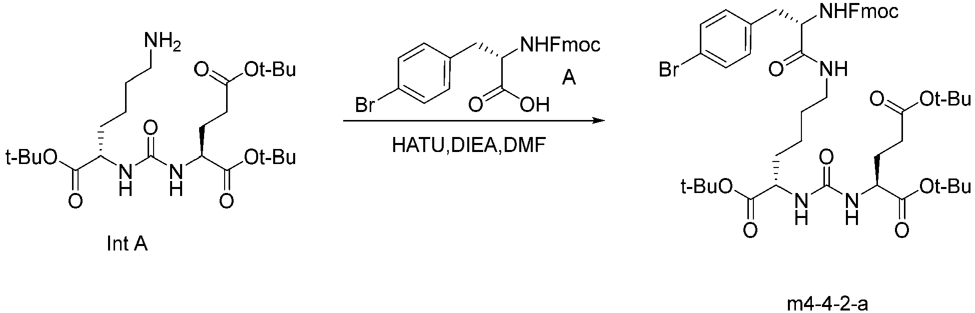

At room temperature, raw material IntA (6.0 g, 12.3 mmol, 1.0 eq.) was dissolved in DMF (60 mL) in a 250 mL single-neck flask, and raw material A (6.9 g, 14.8 mmol, 1.2 eq.), DIEA (2.4 g, 18.5 mmol, 1.5 eq.) and HATU (5.6 g, 14.8 mmol, 1.2 eq.) were separately added thereto. A resulting mixture was stirred at room temperature overnight. Upon product formation as monitored by LCMS, the reaction was stopped. Water and ethyl acetate were added and subjected to extraction and liquid layering. A resulting organic phase was washed with saturated brine, and after separation, a resulting organic phase was separated and concentrated. A resulting crude product was purified by silica gel column chromatography (DCM : MeOH = 50 : 1-2 : 1) to yield product m4-4-2-a (13 g).

### Step 2: Synthesis of compound m4-4-2-b

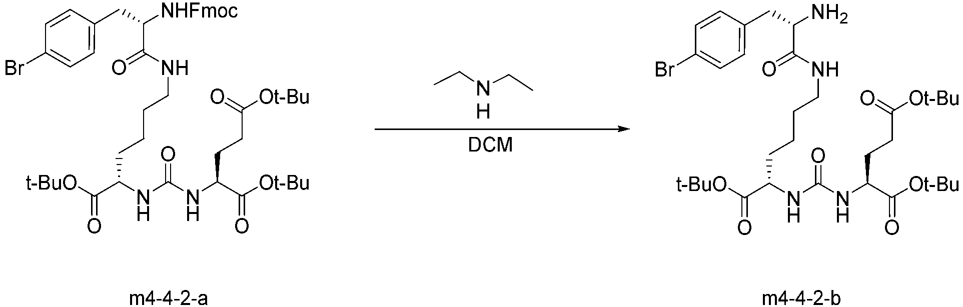

At room temperature, in a 100 mL single-neck flask, raw material m4-4-2-a (12.0 g, 12.8 mmol, 1.0 eq.) was added, followed by adding DCM (120 mL), and the m4-4-2-a was dissolved by stirring. Diethylamine (9.4 g, 128 mmol, 10.0 eq.) was then added thereto and a resulting mixture was stirred at 25°C overnight. Upon product formation and complete consumption of the raw material as monitored by LCMS, the reaction was stopped. A resulting reaction solution was concentrated to dryness to yield a crude product. The crude product was purified by silica gel column chromatography (DCM : MeOH = 50 : 1-10 : 1) to yield product m4-4-2-b (7.0 g) as a pale yellow viscous liquid.

### Step 3: Synthesis of compound m4-4-2-c

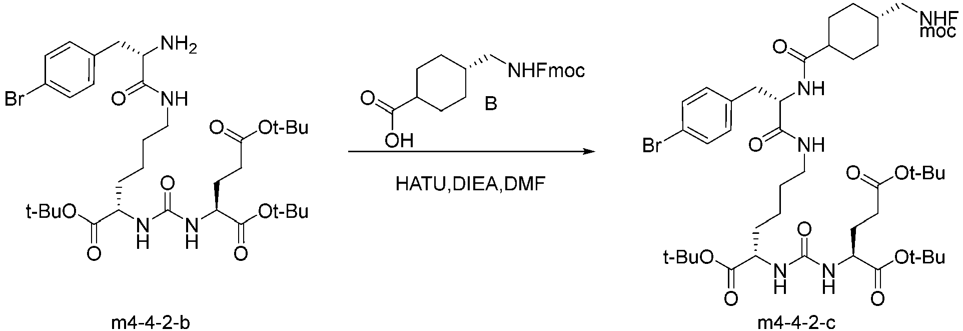

At room temperature, raw material m4-4-2-b (4.0 g, 5.6 mmol, 1.0 eq.) was dissolved in DMF (60 mL) in a 100 mL single-neck flask, and raw material B (2.2 g, 5.6 mmol, 1.0 eq.), DIEA (1.1 g, 8.4 mmol, 1.5 eq.) and HATU (2.6 g, 6.7 mmol, 1.2 eq.) were separately added thereto. A resulting mixture was stirred at room temperature overnight. Upon product formation as monitored by LCMS, the reaction was stopped. Water and ethyl acetate were added thereto and subjected to extraction and liquid layering. A resulting organic phase was washed with saturated brine, and after separation, a resulting organic phase was concentrated. A resulting crude product was purified by silica gel column chromatography (DCM : MeOH = 80 : 1-10 : 1) to yield product m4-4-2-c (4.5 g).

### Step 4: Synthesis of compound m4-4-2-d

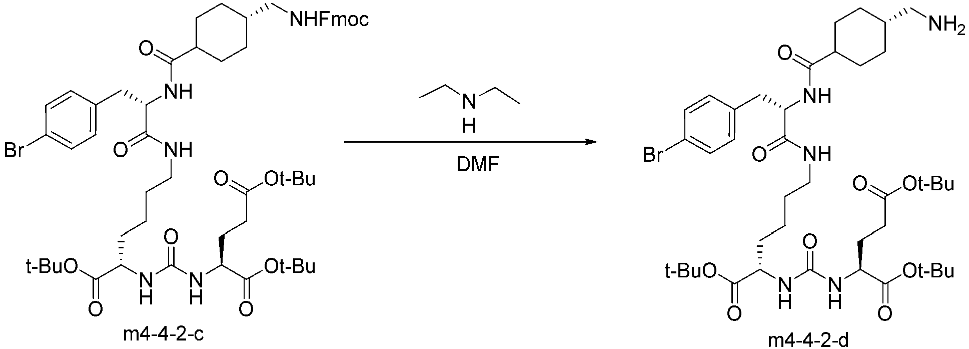

At room temperature, in a 100 mL single-neck flask, raw material m4-4-2-c (4.5 g, 4.2 mmol, 1.0 eq.) was added, followed by adding DCM (60 mL), and the m4-4-2-c was dissolved by stirring. Diethylamine (3.1 g, 42 mmol, 10.0 eq.) was added thereto and a resulting mixture was stirred at 25°C overnight. Upon product formation and complete consumption of the raw material as monitored by LCMS, the reaction was stopped. A resulting reaction solution was concentrated to dryness to yield a crude product. The crude product was purified by silica gel column chromatography (DCM : MeOH = 50 : 1-10 : 1) to yield product m4-4-2-d (1.8 g) as a pale yellow viscous liquid.

### Step 5: Synthesis of compound m4-4-2-e

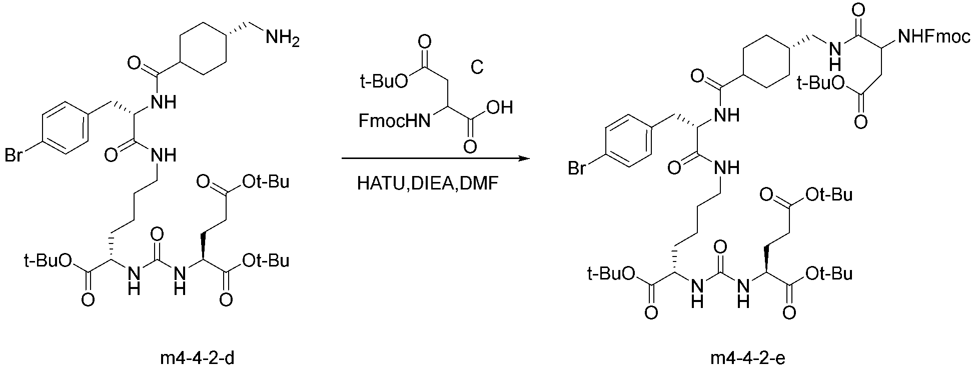

At room temperature, raw material m4-4-2-d (1.6 g, 1.9 mmol, 1.0 eq.) was dissolved in DMF (20 mL) in a 100 mL single-neck flask, and raw material C (0.93 g, 2.3 mmol, 1.2 eq.), DIEA (0.36 g, 2.8 mmol, 1.5 eq.) and HATU (0.86 g, 2.3 mmol, 1.2 eq.) were separately added. A resulting mixture was stirred at room temperature overnight. Upon product formation as monitored by LCMS, the reaction was stopped. Water and ethyl acetate were added and subjected to extraction and liquid layering. A resulting organic phase was washed with saturated brine, and after separation, a resulting organic phase was concentrated. A resulting crude product was purified by silica gel column chromatography (DCM : MeOH = 80 : 1-10 : 1) to yield product m4-4-2-e (1.8 g).

### Step 6: Synthesis of compound m4-4-2-f

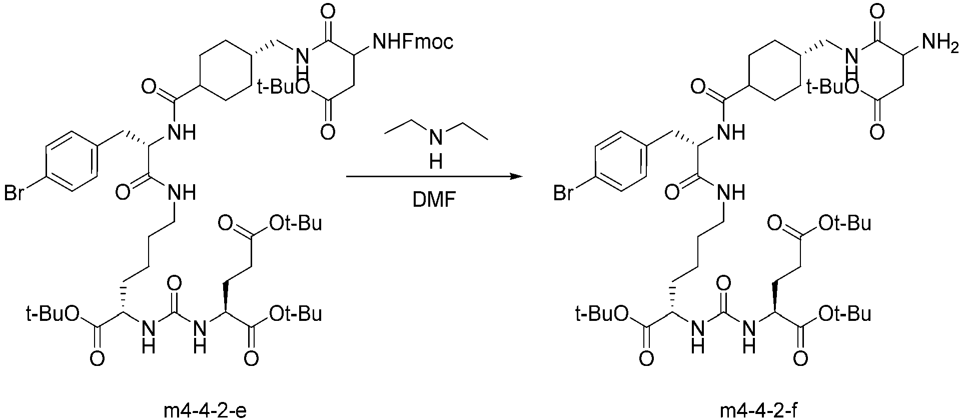

At room temperature, in a 50 mL single-neck flask, raw material m4-4-2-e (1.8 g, 1.4 mmol, 1.0 eq.) was added, followed by adding DCM (20 mL), and the m4-4-2-e was dissolved by stirring. Diethylamine (1.1 g, 14 mmol, 10.0 eq.) was then added thereto and a resulting mixture was stirred at 25°C overnight. Upon product formation and complete consumption of the raw material as monitored by LCMS, the reaction was stopped. A resulting reaction solution was concentrated to dryness to yield a crude product. The crude product was purified by silica gel column chromatography (DCM : MeOH = 50 : 1-10 : 1) to yield product m4-4-2-f (1.3 g) as a pale yellow viscous liquid.

### Step 7: Synthesis of compound m4-4-2-g

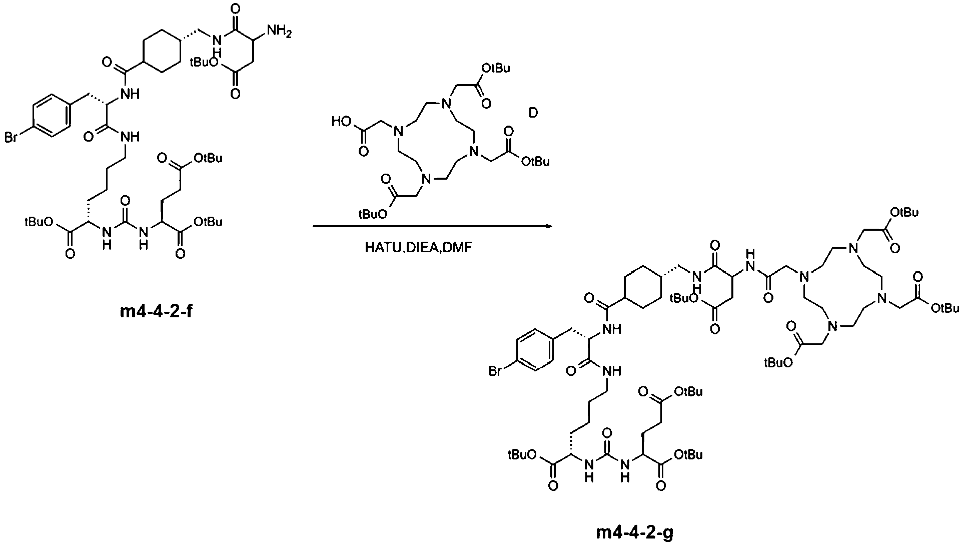

At room temperature, raw material m4-4-2-f (1.2 g, 1.2 mmol, 1.0 eq.) is dissolved in DMF (25 mL) in a 50 mL single-neck flask. Tri-tert-butyl 1,4,7,10-tetraazacyclododecane-1,4,7,10-tetraacetic acid (0.81 g, 1.4 mmol, 1.2 eq.), DIEA (0.3 g, 2.3 mmol, 2.0 eq.) and HATU (0.53 g, 1.4 mmol, 1.2 eq.) were separately added and a resulting mixture was stirred at room temperature overnight. Upon product formation as monitored by LCMS, the reaction was stopped. Water and ethyl acetate were added thereto and subjected to extraction and liquid layering. A resulting organic phase was washed with saturated brine, and after separation, a resulting organic phase was concentrated. A resulting crude product was purified by silica gel column chromatography (DCM : MeOH = 80 : 1-10 : 1) to yield product m4-4-2-g (0.95 g).

### Step 8: Synthesis of compound m4-4-2

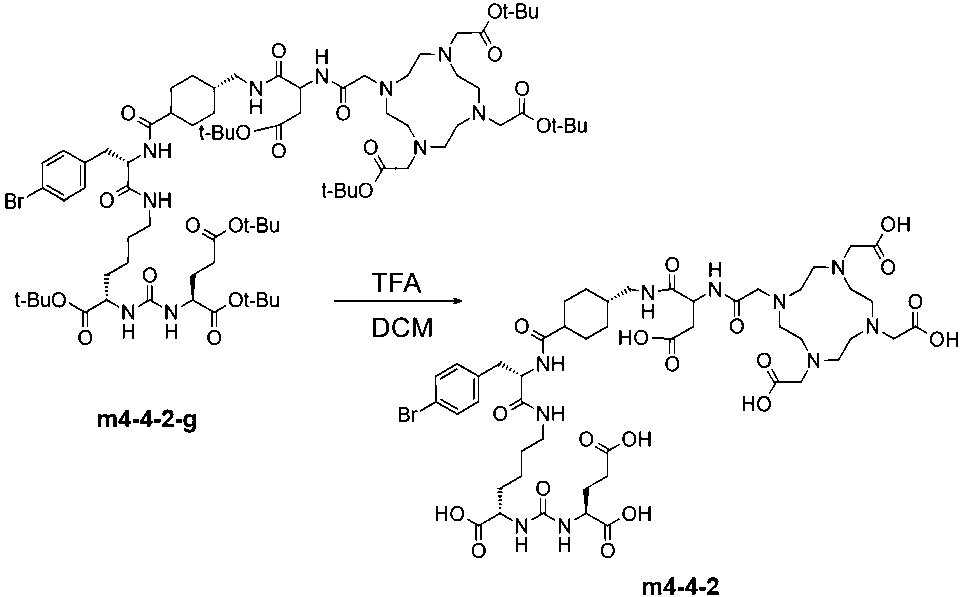

At room temperature, in a 50 mL single-neck flask, raw material m4-4-2-g (0.6 g, 0.38 mmol, 1.0 eq.) was added, followed by adding DCM (10 mL), and the m4-4-2-g was dissolved by stirring. TFA (2.2 g, 19.0 mmol, 50.0 eq.) was added and a resulting mixture was reacted at 25°C overnight. Upon product formation and complete consumption of the raw material as monitored by LCMS, the reaction was stopped. A resulting organic phase was concentrated under reduced pressure and then purified by preparative high-performance liquid phase chromatography (Welch Xtimate C18, 21.2 × 250 mm, 5 µm) to yield m4-4-2 (230 mg, with a yield of 51%, and a purity of 97.05% ) as a white solid.

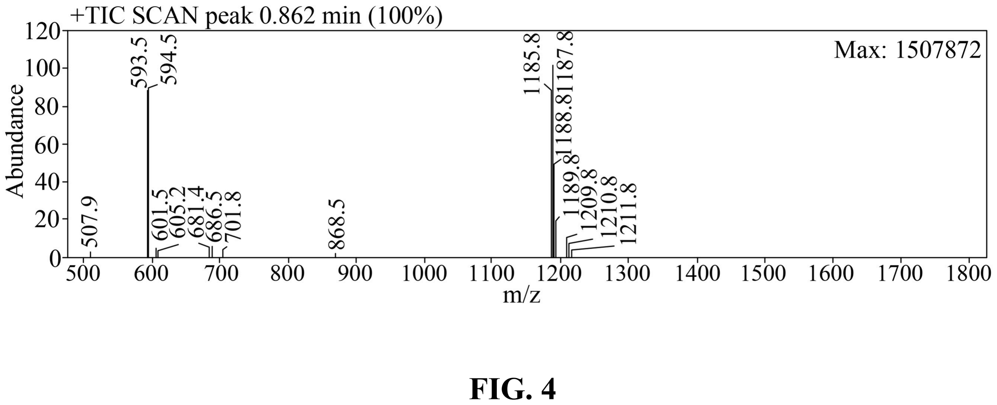

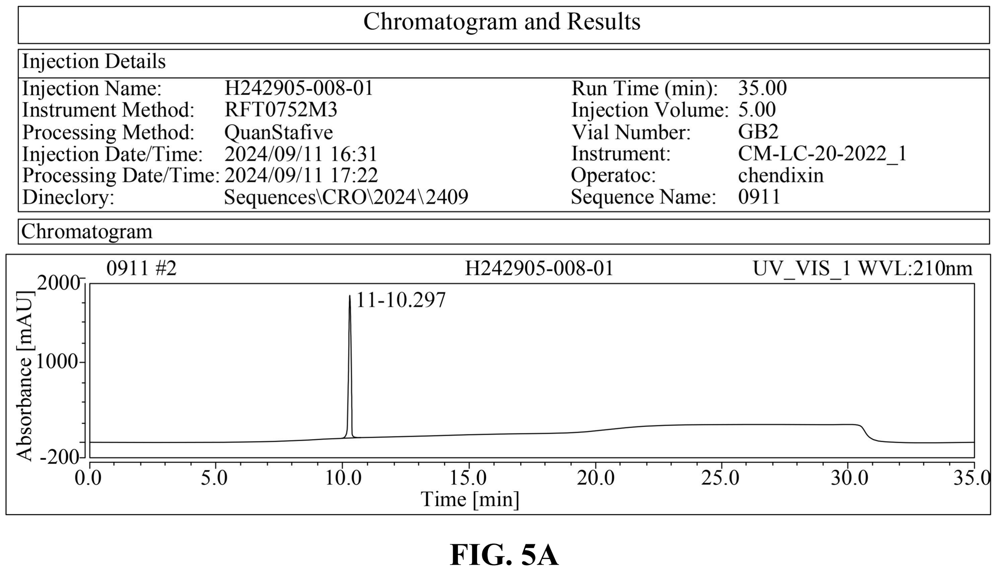

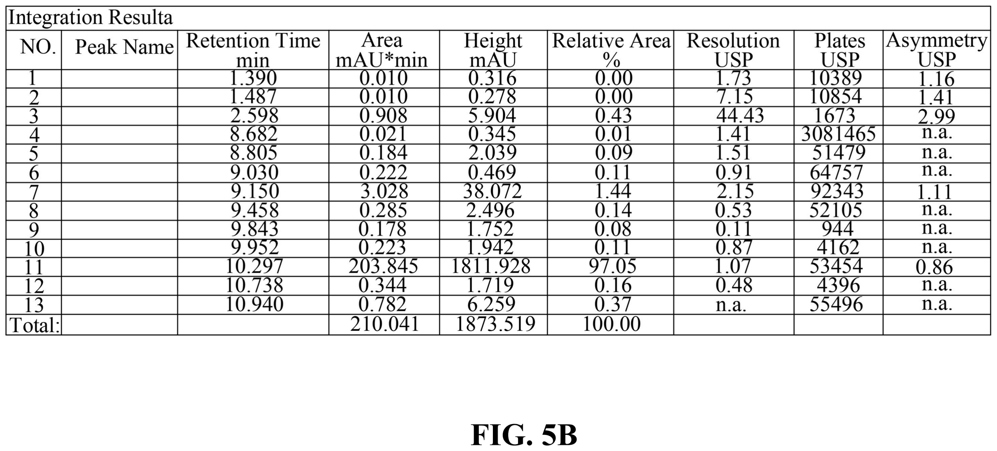

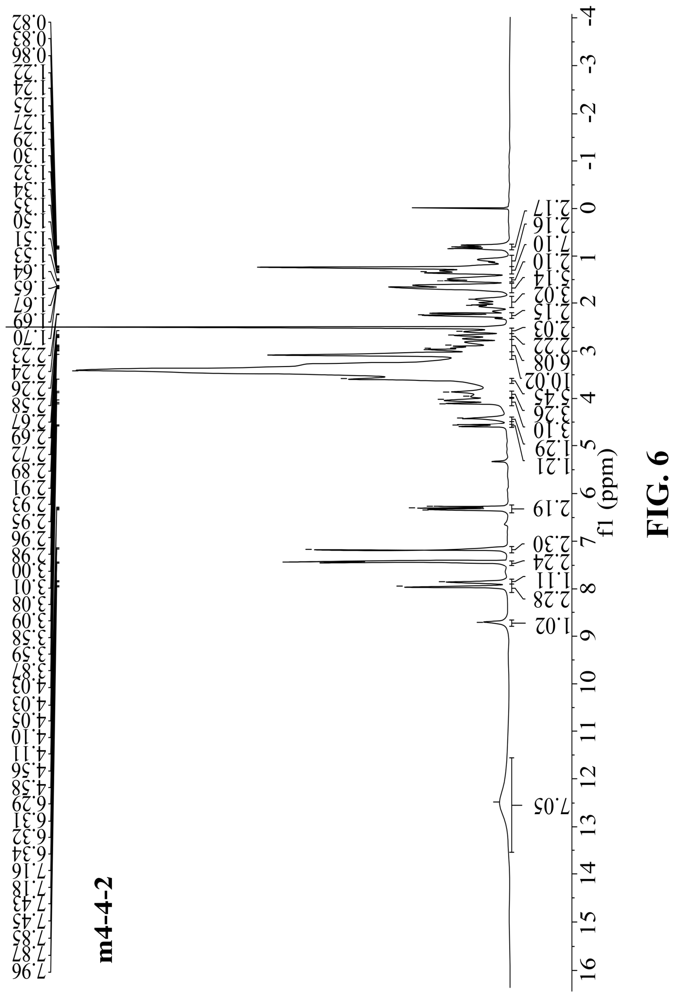

The LC-MS, HPLC and ¹H-NMR spectra of m4-4-2 are shown in FIG. 4, FIG. 5A and FIG. 6, respectively.

### Example 3 Biological activity test

### 1. Log P value

The Log P values of the test compounds and control compounds in 1-octanol/PBS (pH 1.0) were determined using the shake flask method. The results are shown in Tables 1-2.

### 1.1 Experimental reagents:

100 mM Sodium phosphate buffer (pH = 1.0), and n-octanol.

### 1.2 Experimental method:

(1) To a 96-well deep-well plate, 15 µL of a stock solution (30 mM) of each test compound or control compound prepared in DMSO was added. Blank dimethyl sulfoxide was then added to bring the total volume to 45 µL, resulting in a working concentration of 10 mM for each test compound or control compound.
(2) 15 µL of the 10 mM working solutions were sequentially transferred into respective 96-well deep-well plates (LogP plate). To each vial of the LogP plate, 800 µL of saturated n-octanol was added, followed by adding 200 µL of saturated phosphate buffer. The LogP plate was sealed with a 96-well plate cover, transferred to an Eppendorf Thermomixer Comfort plate shaker, and shaken at 2000 rpm at 25°C for 2 h.
(3) The samples were centrifuged at 3220 g at 25°C for 30 min for phase separation. Using a pipette and syringe, about 100 µL of each phase (the n-octanol phase and the buffer phase) was transferred to a new 96-well plate. 5 µL of the n-octanol sample was transferred to a new 96-well plate, followed by adding 495 µL of a mixture of acetonitrile : H₂O (1 : 1) to obtain a 100-fold diluted n-octanol sample. The sample was eddied at 1000 rpm for 5 min.
(4) 50 µL of the 100-fold diluted n-octanol sample was transferred to a new 96-well plate, followed by adding 450 µL of a mixture of acetonitrile : H₂O (1 : 1) containing an internal standard, yielding a 1000-fold diluted n-octanol sample. The sample was eddied at 1000 rpm for 5 min.
(5) 50 µL of the buffer sample was transferred to a new 96-well plate, followed by adding 450 µL of a mixture of acetonitrile : H₂O (1 : 1) containing an internal standard, yielding a 10-fold diluted buffer sample. The sample was eddied at 1000 rpm for 5 min.
(6) The samples were analyzed by LC-MS/MS.

### 1.3 Data analysis:

The LogP for the compound was calculated according to the following equation: $LogP = Log \left[\left({AR}_{Oct}\times DF\right)/\left({AR}_{Buffer}\times DF\right),\right.$ where AR_{Oct} refers to the peak area ratio of the test compound in the n-octanol phase; AR_{Buffer} refers to the peak area ratio of the test compound in the buffer phase; and DF refers to dilution fold.

**Table 1. LogP measurements of test compounds and control compounds**

| **Compound** | **LogP** |
|---|---|
| Nicardipine | 1.16 |
| Progesterone | 3.71 |
| PSMA-617 | < -4.02 |
| m4-4 | < -3.88 |

When the test compound was not detected in the octanol phase (below the limit of detection), the limit of detection value was used as the peak area for cut-off value calculation.

**Table 2. LogP measurements of test compounds and control compounds**

| **Compound** | **LogP** |
|---|---|
| Nicardipine | 1.35 |
| Progesterone | 3.79 |
| PSMA-617 | < -3.55 |
| m4-4-2 | < -2.89 |

When the test compound was not detected in the octanol phase (below the limit of detection), the limit of detection value was used as the peak area for cut-off value calculation.

The LogP test results of two batches recorded in Tables 1-2 are shown below:
PSMA-617 (< -4.02) vs m4-4 (< -3.88);
PSMA-617 (< -3.55) vs m4-4-2 (< -2.89).

The test results for the compounds of the present disclosure in each batch are at the same level as those for PSMA-617 (i.e., within three times the systematic error), indicating that they achieve logP properties similar to PSMA-617. This provides a basis for proceeding with subsequent biological tests.

### 2. Enzymatic affinity

The binding of the test compounds and control compounds to PSMA was determined using a fluorometric assay. The results are shown in Tables 3-4.

### 2.1 Experimental reagents:

PSMA protein, NAAG substrate, OPA.

### 2.2 Experimental method:

(1) To a 384-well dilution plate, 60 µL of a 10 µM solution of a test compound or a control compound was added. The solution was then serially diluted with DMSO at ratio of 1 : 3 to generate solutions with 10 different concentrations.
(2) 0.1 µL of the solutions with different concentrations were transferred to a 384-well assay plate, with two wells replicating for per concentration.
(3) 5 µL of the PSMA enzyme working solution (10 nM) was added to the 384-well plate. The plate was centrifuged at 1000 rpm for 1 minute and incubated at 25°C for 10 min.
(4) The reaction was initiated by adding 5 µL of the NAAG substrate working solution (10 µM). The plate was centrifuged at 1000 rpm for 1 minute and incubated at 25°C for 60 min.
(5) The reaction was initiated by adding 5 µL of the OPA working solution (4 mg/mL). The plate was centrifuged at 1000 rpm for 1 minute and incubated at 25°C for 15 min.
(6) Fluorescence signals were read using a BMG plate reader at Ex 330 nm and Em 450 nm.

### 2.3 Data analysis:

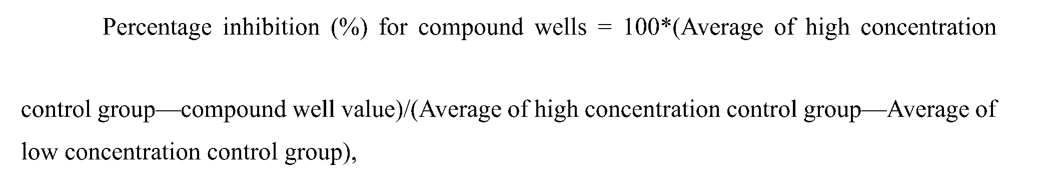

Percentage inhibition (%) for compound wells = 100*(Average of high concentration control group-compound well value)/(Average of high concentration control group-Average of low concentration control group),
High concentration control group: DMSO and enzyme;
Low concentration control group: DMSO and buffer; $S / B = Averange of high concentration control group / Averange of low concentration control group ;$ 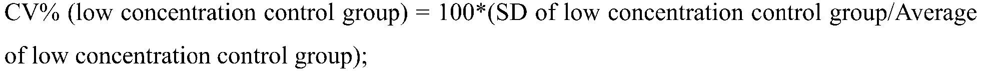 CV% (low concentration control group) = 100*(SD of low concentration control group/Average of low concentration control group); 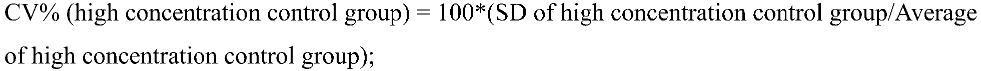 CV% (high concentration control group) = 100*(SD of high concentration control group/Average of high concentration control group); 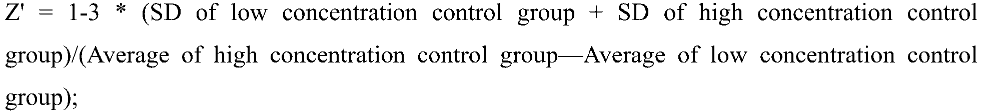 Z' = 1-3 * (SD of low concentration control group + SD of high concentration control group)/(Average of high concentration control group-Average of low concentration control group);
the IC₅₀ values were fitted using a nonlinear regression equation with XLfit 5.5.0: $Y = Bottom + \left(Top-Bottom\right) \left(1+{10}^{∧}\left(\left({LogIC}_{50}-X\right)*HillSlope\right)\right) ,$
where X: Logarithm of the compound concentration;
Y: Percentage inhibition (%);
Top and Bottom refer to fluorescence value of the high concentration control group and fluorescence value of the low concentration control group, respectively (expressed in the same unit as Y);
logIC₅₀: in the same logarithmic unit as X; and
HillSlope: slope factor or slope.

**Table 3. Test results of the inhibition of test compounds and control compounds against PSMA**

| **Compound** | **30 min** | | | | |
|---|---|---|---|---|---|
| | **Maximum inhibition%** | **Relative value** | **Absolute value** | | |
| | | **IC₅₀ (nM)** | **IC₅₀ (nM)** | **IC₈₀ (nM)** | **IC₉₀ (nM)** |
| PSMA-617 | 103.54 | 3.12 | 2.34 | 7.99 | 14.20 |
| PSMA-617-HG | 102.23 | 16.41 | 14.99 | 23.68 | 29.82 |
| m4-4 | 104.04 | 3.36 | 2.98 | 5.25 | 6.92 |

**Table 4. Test results of the inhibition of test compounds and control compounds against PSMA**

| **Compound** | **Bottom** | **Top** | **IC₅₀ (nM)** | **Slope** |
|---|---|---|---|---|
| PSMA-617 | -8.48 | 94.4 | 2.45 | 2.14 |
| PSMA-617-HG | -6.71 | 94.5 | 2.84 | 2.04 |
| m4-4-2 | -0.30 | 93.4 | 2.74 | 2.68 |

The enzymatic experimental results of the compound of the present disclosure and PSMA-617 are as follows:
PSMA-617 (3.12 nM) vs m4-4 (3.36 nM);
PSMA-617 (2.45 nM) vs m4-4-2 (2.74 nM).

The results from the aforementioned two batches demonstrate that the compound of the present disclosure exhibits an enzymatic affinity for PSMA that is at a comparable level to that of the control compound PSMA-617, with all data falling within three times the systematic error. This provides a foundation for the subsequent screening of compounds with superior uptake and internalization properties.

### 3. Cellular uptake

Cellular uptake experiments were carried out for the test compounds and control compounds using LNCap cells. The results are shown in Tables 5-7.

### 3.1 Experimental reagents:

Phenol red-free RPMI 1640 medium, 10% FBS, Glu-490 probe, PBS buffer, 0.25% trypsin without EDTA.

### 3.2 Experimental method:

(1) Cells were resuspended in phenol red-free RPMI 1640 medium containing 10% FBS in a 48-well plate and then cultured at 37°C with 5% CO₂ for 24 h.
(2) Test compounds were added to the cells and the plate was incubated at 4°C for 30 min.
(3) Glu-490 was added to the cells to a final concentration of 0.3 µM and the plate was incubated at 4°C for 1 h.
(4) The cells were rinsed with pre-chilled PBS at 4°C.
(5) The cells were collected using 0.25% trypsin without EDTA, and centrifuged at 400 g for 5 min, and the supernatant was discarded.
(6) The cells were resuspended in 100 µL of PBS without washing, and the sample was detected using a CytoFLEXS flow cytometer (Beckman). Analysis was performed using ModFit software.

### 3.3 Data analysis:

H = Ave (DMSO);
L = Ave (without Glu490);
SD (H) = STDEV (DMSO);
SD (L) = STDEV (without Glu490); $CV % \left(DMSO\right) = 100 * \left(SD_H/Ave_H\right) ;$ $CV % \left(without Glu490\right) = 100 * \left(SD_L/Ave_L\right) ;$ $Z ′ = 1 - 3 * \left(SD_H+SD_L\right) / \left(Ave_H-Ave_L\right) ;$ $Inhibition % = \left(sample-Ave_L\right) / \left(Ave_H-Ave_L\right) ;$
the IC₅₀ values were fitted using a nonlinear regression equation: $Y = Bottom + \left(Top-Bottom\right) / \left(1+{10}^{∧}\left(\left(LogIC50-X\right)*HillSlope\right)\right) ;$
X: Concentration,
Y: Inhibition rate,
Top and Bottom refer to the fluorescence values corresponding to H = Ave (DMSO) and L = Ave (without Glu490), respectively,
HillSlope: Slope factor or slope.

**Table 5. Uptake results of test compounds and control compounds in LNCap cells**

| **Compound** | **Initial concentration (nM)** | **Dilution fold** | **Minimum inhibition%** | **Maximum inhibition%** | **Slope** | **R_IC₅₀ (nM)** | **A_IC₅₀ (nM)** |
|---|---|---|---|---|---|---|---|
| PSMA-617 | 10000 | 5 | 8.90 | 88.84 | 0.47 | 4.16 | 7.15 |
| m4-4 | 10000 | 5 | 5.38 | 87.61 | 0.57 | 2.17 | 4.90 |

**Table 6. Uptake results of test compounds and control compounds in LNCap cells**

| **Compound** | **Parallel 1** | **Parallel 2** | **4 h uptake** |
|---|---|---|---|
| PSMA-617 | 4.35% | 5.98% | 5.17% ± 1.15% |
| m4-4 | 7.48% | 6.58% | 7.03% ± 0.63% |

**Table 7. Uptake results of test compounds and control compounds in LNCap cells**

| **Compound** | **Initial concentration (nM)** | **Dilution fold** | **Minimum inhibition%** | **Maximum inhibition%** | **Slope** | **R_IC₅₀ (nM)** | **A_IC₅₀ (nM)** |
|---|---|---|---|---|---|---|---|
| PSMA-617 | 10000 | 4 | 6.00 | 76.30 | 0.37 | 9.42 | 0.36 |
| m4-4-2 | 10000 | 4 | -11.38 | 60.61 | 0.61 | 1077.21 | 2779.34 |

The IC₅₀ of the uptake of the compounds of the present disclosure at multiple concentration points was tested using the FACS method. Results are as follows:
PSMA-617 (4.16 nM) vs m4-4-2 (2.17 nM), PSMA-617 (9.42 nM) vs m4-4-2 (1077.21 nM).

The 4 h uptake was tested by the LC-MS/MS method. Results are as follows:
PSMA-617 (5.17%) vs m4-4 (7.03%).

The IC₅₀ results of the uptake of the compounds of the present disclosure at multiple concentration points tested using the FACS method, and the 4 h uptake results tested by the LC-MS/MS method show that: the uptake rate of m4-4 is at the same level as that of the control compound PSMA-617, but better than that of m4-4-2.

### 4. Cellular internalization

Cellular internalization experiments of the test compounds and control compounds were carried out using LNCap cells. The results are shown in Tables 8-9.

### 4.1 Experimental reagents:

HHBS buffer, Phenol red-free RPMI 1640 medium, 10% FBS, PBS buffer, 0.25% trypsin without EDTA.

### 4.2 Experimental method:

LNCap cells in the logarithmic growth phase were taken, digested and centrifuged, and resuspended in HHBS buffer (1 × 10⁶ cells/mL). 200 µL of the cell suspension was then separately placed into each EP tube. The drug (final concentration: 1 µM) and the antibody (200×) were separately added to the tubes, followed by co-incubation at 37°C for 2 h. After incubation, the cells were washed by adding pre-chilled HHBS buffer and centrifuged. An acid wash solution pre-chilled at 4°C was then added for washing and centrifugation. The cells were then fixed with paraformaldehyde at 4°C for 30 min, centrifuged, and washed once with HHBS buffer. After centrifugation, the cells were resuspended in HHBS buffer for flow cytometry.

### 4.3 Data processing:

$Cellular internalization % = \left[1-\left(\left(A2-A0\right)/\left(A1-A0\right)\right)\right] \times 100 % ,$
A0: Background values of cells without antibody or compound,
A1: Fluorescence values after incubation with antibody only,
A2: Fluorescence values after co-incubation with both antibody and compound.

**Table 8. Internalization results of test compounds and control compounds in LNCap cells**

| **Sample name** | **Geometric mean (Alexa Fluor 488-A :: null)** | | **Internalization rate** | **Average** |
|---|---|---|---|---|
| Blank parallel 1 | 57.4 | | / | |
| Blank parallel 2 | 50 | 53.7 | / | |
| BC parallel 1 | 1602 | | -0.78% | 0.00% |
| BC parallel 2 | 1578 | 1590 | 0.78% | |
| PSMA-617 parallel 1 | 746 | | 54.94% | 58.29% |
| PSMA-617 parallel 2 | 643 | | 61.64% | |
| m4-4-2 parallel 1 | 627 | | 62.68% | 58.91% |
| m4-4-2 parallel 2 | 743 | | 55.13% | |

**Table 9. Internalization results of test compounds and control compounds in LNCap cells**

| **Sample name** | **Geometric mean (Alexa Fluor 488-A :: null)** | **Internalization rate** | **Average** |
|---|---|---|---|
| BC 2 h parallel 1 | 30.9 | / | / |
| BC 2 h parallel 2 | 33.5 | / | / |
| BC 24 h | 47 | / | / |
| PSMA-617 2 h parallel 1 | 20.2 | 34.63% | 39.25% |
| PSMA-617 2 h parallel 2 | 18.8 | 43.88% | |
| PSMA-617 24 h parallel 1 | 45.6 | 2.98% | -0.32% |
| PSMA-617 24 h parallel 2 | 48.7 | -3.62% | |
| m4-4 2 h parallel 1 | 20.9 | 32.38% | 38.27% |
| m4-4 2 h parallel 2 | 18.7 | 44.18% | |
| m4-4 24 h parallel 1 | 43 | 8.51% | 9.57% |
| m4-4 24 h parallel 2 | 42 | 10.64% | |

Result comparisons of the internalization rate experiment between the compounds of the present application and the control compound PSMA-617 show:
PSMA-617 (58.29%) vs m4-4-2 (58.91%);
PSMA-617 (39.25%) vs m4-4 (38.27%).

Comparisons of internalization rates between the compounds of the present disclosure across multiple batches and the control compound PSMA-617 demonstrate that the internalization rates of m4-4, m4-4-2 and the control compound are comparable.

### 5. Plasma stability

The stability of compounds PSMA-617, m4-4 and m4-4-2 in mouse and human plasma was investigated. The results are shown in Tables 10-11.

### 5.1 Experimental reagents:

### Plasma information:

| Matrix type | | Anticoagulant | Source | |
|---|---|---|---|---|
| Mouse | | Heparin sodium | Hunan Everpro Medical Co., Ltd., China Zibo Municipal Hospital | |
| Human | | Heparin sodium | | |
| Species | Cat. No. | Lot. No. | Description | Supplier |
| Human | CMS-S50 | F03010 | Pooled | SHQY |
| Species | Cat. No. | Lot. No. | Description | Supplier |
| Mouse | 0191E1.11 | 23B007 | Pooled | IPHASE |

### 5.2 Experimental method:

An appropriate amount of blank plasma was added to an EP tube. A working solution of the test substance (final concentration: 0.5 µM) was then added thereto and incubated, followed by vortex mixing. The samples were incubated in a water bath at 37°C with two paralleling groups, with incubation time points of 0, 2, 4, and 24 h. After incubation, sample preprocessing was performed: 20 µL of the sample was collected from the incubation system, and 250 µL of an internal standard precipitant was added; a resulting mixture was vortex-mixed, followed by adding 100 µL of ultrapure water. The samples were centrifuged (5500 g) for 10 min, and 250 µL of a resulting supernatant was transferred to a 96-well plate, vortex-mixed, and analyzed by LC-MS/MS.

### 5.3 Data processing:

$Remaining original drug % = At / A 0 \times 100 %$
At: Amount of the test substance at the incubation time point;
A0: Amount of the test substance in 0 h sample; and
T1/2 = 0.693/k.

**Table 10. Stability results of compounds PSMA-617 and m4-4 in mouse and human plasma**

| **Compound** | **Mouse** | | **Human** | |
|---|---|---|---|---|
| | **Remaining amount (%)** | **t1/2 (h)** | **Remaining amount (%)** | **t1/2 (h)** |
| Propantheline | 0.175 | 0.675 | 0.0440 | 0.525 |
| PSMA-617 | 105 | > 48* | 100 | > 48* |
| m4-4 | 117 | > 48* | 94.4 | > 48* |

| | | | | |
|---|---|---|---|---|
| * After 24 h of incubation in the matrix, not less than 75% of the compound is remained, which by default indicates t_{1/2} > 48 h. This means that at 24 h, 75% of the compound is remained, and at 48 hours, 50% of the compound is remained (t_{1/2}). | | | | |

**Table 11. Stability results of compounds PSMA-617 and m4-4-2 in mouse and human plasma**

| **Compound** | **Mouse** | | **Human** | |
|---|---|---|---|---|
| | **Remaining amount (%)** | **t1/2 (h)** | **Remaining amount (%)** | **t1/2 (h)** |
| Propantheline | 0.0405 | 0.361 | 0.0319 | 0.339 |
| PSMA-617 | 95.2 | > 48* | 85.7 | > 48* |
| m4-4-2 | 92.3 | > 48* | 108 | > 48* |

| | | | | |
|---|---|---|---|---|
| *After 24 h of incubation in the matrix, not less than 75% of the compound is remained, which by default indicates t_{1/2} > 48 h. This means that at 24 h, 75% of the compound is remained, and at 48 hours, 50% of the compound is remained (t_{1/2}). | | | | |

Results of plasma protein stability experiments are as follows:
PSMA-617 > 48 h (mouse) or > 48 h (human) vs m4-4 > 48 h (mouse) or > 48 h (human), PSMA-617 > 48 h (mouse) or > 48 h (human) vs m4-4-2 > 48 h (mouse) or > 48 h (human).

Based on the plasma protein stability experiment results presented above, the compounds of the present disclosure exhibit stability in plasma at the same level to that of the control compound. The plasma protein binding rate of the compounds of the present disclosure was further evaluated.

### 6. Plasma protein binding rate

The binding rates of compounds PSMA-617, m4-4 and m4-4-2 to mouse and human plasma proteins were studied. Results are shown in Tables 12-13. In the experiment, ${f}_{u} = \frac{{C}_{R}}{{C}_{D}} \times 100 % , {f}_{b} = \left(1-{f}_{u}\right) \times 100 % , Recovery rate = \frac{{C}_{R} + {C}_{D}}{{C}_{I}} \times 100 % .$

### 6.1 Experimental reagents:

| Matrix type | | Anticoagulant | | |
|---|---|---|---|---|
| Mouse plasma | | EDTA-2K | | |
| Human plasma | | EDTA-2K | | |
| Species | Cat. No. | Lot. No. | Strain and gender | Supplier |
| Mouse | 0184E1.11 | B0124H0009 | Mixed gender | IPHASE |
| Species | Cat. No. | Lot. No. | Strain and gender | Supplier |
| Human | CMS-S50 | F03010 | Pooled | SHQY |

### 6.2 Experimental method:

The test compound was added to plasma pre-warmed to 37°C to achieve a final concentration of 0.5 µM. Pre-treated dialysis membranes were assembled into a dialysis plate. 120 µL of the test compound prepared in plasma was added to the donor side of the dialysis membrane in each dialysis well, and 120 µL of 0.002% Tween-PB was added to the receptor side. A 20 µL sample of the test compound at the final concentration was taken and added to a 96-well sample plate to obtain the T0 sample, and the T0 sample was stored in a refrigerator at -20°C. The remaining sample was incubated with shaking at 37°C for 20 h. After incubation, 20 µL of samples were taken from the post-dialysis receptor side (sample B), post-dialysis donor side (sample A), and stability sample (20 h stability sample), respectively. Corresponding volumes of blank plasma or 0.002% Tween-PB were added to the sample B, sample A, T0 sample, and 20 h stability sample, respectively, to achieve a plasma-to-buffer volume ratio of 1 : 1 in each sample well. To all sample wells of the test substance group, 250 µL of precipitant containing internal standard was added, followed by adding 80 µL of ultrapure water. The mixture was mixed uniformly and then centrifuged, and 250 µL of a resulting supernatant was collected for a second centrifugation prior to injection and analysis.

### 6.3 Data analysis:

The percentage of unbound compound in plasma and the recovery rate were calculated according to the following equations: $Plasma protein binding rate \left(%\right) = 1 - Percentage of unbound compound ;$ $Percentage of unbound compound \left(%\right) = CB / CA ;$ $Recovery rate \left(%\right) = \left(CB+CA\right) / CT 0 ;$
where CB is the concentration of the compound in the receptor solution after equilibrium dialysis;
CA is the concentration of the compound in the donor plasma after equilibrium dialysis; and
CT0 is the initial concentration of the compound in plasma.

**Table 12. Protein binding rate of compounds PSMA-617 and m4-4 in mouse and human plasma**

| **Compound** | **Concentration (µM)** | **Species** | **Results** | | | |
|---|---|---|---|---|---|---|
| | | | **Fᵤ (%)** | **F_{b} (%)** | **Recovery rate (%)** | **Average stability (%)** |
| Warfarin | 2 | Mouse | 7.70 | 92.3 | 99.1 | NA |
| PSMA-617 | 0.5 | Mouse | 78.7 | 21.3 | 89.6 | 91.7 |
| m4-4 | 0.5 | Mouse | 81.6 | 18.4 | 95.1 | 104 |
| Warfarin | 2 | Human | 0.609 | 99.4 | 94.8 | NA |
| PSMA-617 | 0.5 | Human | 14.0 | 86.0 | 91.7 | 105 |
| m4-4 | 0.5 | Human | 17.4 | 82.6 | 90.8 | 110 |

**Table 13. Protein binding rate of compounds PSMA-617 and m4-4-2 in mouse and human plasma**

| **Compound** | **Concentration (µM)** | **Species** | **Results** | | | |
|---|---|---|---|---|---|---|
| | | | **Fᵤ (%)** | **F_{b} (%)** | **Recovery rate (%)** | **Average stability (%)** |
| Warfarin | 2 | Mouse | 16.5 | 83.5 | 89.5 | NA |
| PSMA-617 | 0.5 | Mouse | 82.1 | 17.9 | 87.0 | 86.2 |
| m4-4-2 | 0.5 | Mouse | 92.2 | 7.79 | 99.0 | 104 |
| Warfarin | 2 | Human | 0.558 | 99.4 | 90.0 | NA |
| PSMA-617 | 0.5 | Human | 14.8 | 85.2 | 90.4 | 97.5 |
| m4-4-2 | 0.5 | Human | 50.6 | 49.4 | 109 | 98.0 |

The plasma protein binding rates serve as reference for subsequent *in vivo* and *in vitro* metabolism experiments, with the human plasma protein binding rate with the compound being the primary reference standard. Experimental results are as follows:
PSMA-617 (86.0%) vs m4-4 (82.6%);
PSMA-617 (85.2%) vs m4-4-2 (49.4%).

The results show that m4-4 and the control compound have the same level of human plasma protein binding rates, with both falling within the acceptable range, whereas m4-4-2 shows a lower human plasma protein binding rate.

### 7. Liver microsomal stability

The stability of compounds PSMA-617 and m4-4 was investigated in mouse, rat, and human liver microsomes. The results are shown in Tables 14-15. In the experiment, CLint (mic) represents intrinsic clearance in microsomes, R2 represents linear correlation coefficient, and T1/2 represents half-life.

### 7.1 Experimental reagents:

### Liver microsome information

| Species | Cat. No. | Lot. No. | Strain and gender | Supplier |
|---|---|---|---|---|
| Human | 452117 | 38300 | Male and female | Gentest |
| Rat | 452501 | 1260001 | Male Sprague Dawley | Gentest |
| Mouse | LMCD-100P | LMCD-2311v211 | Male CD-1 | Milestone |

### 7.2 Experimental method:

Liver microsome (LM) solution (0.5587 mg/mL) was added to a 96-well plate (culture plate), followed by adding NADPH (final concentration: 1 mM) or PBS (negative control). A resulting mixture was mixed at 800 rpm for 10 s and then pre-warmed at 37°C for 10 min. The test compound was added to the culture plate (final concentration: 2 µM), and the reaction was initiated. At incubation time points (0.5, 15, 30, 45, and 60 min), 50 µL of the solution was transferred from the incubation plate to a sample plate filled with 200 µL of pre-chilled methanol containing an internal standard to terminate the reaction. The sample plate was centrifuged at 3220 g for 40 min. 100 µL of the supernatant was transferred to an analysis plate containing an appropriate volume of H₂O for LC-MS/MS analysis.

### 7.3 Data analysis:

(1) The slope k of the straight line (ln(remaining compound percentage) vs culture time) was calculated using Microsoft Excel;
(2) The *in vitro* t_{1/2} and *in vitro* Clint were calculated using the following equations:$T 1 / 2 \left(min\right) = 0.693 / k ;$ 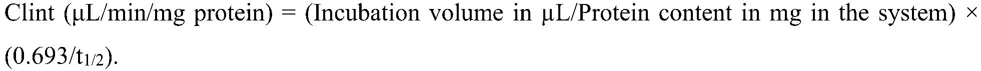 Clint (µL/min/mg protein) = (Incubation volume in µL/Protein content in mg in the system) × (0.693/t1/2).

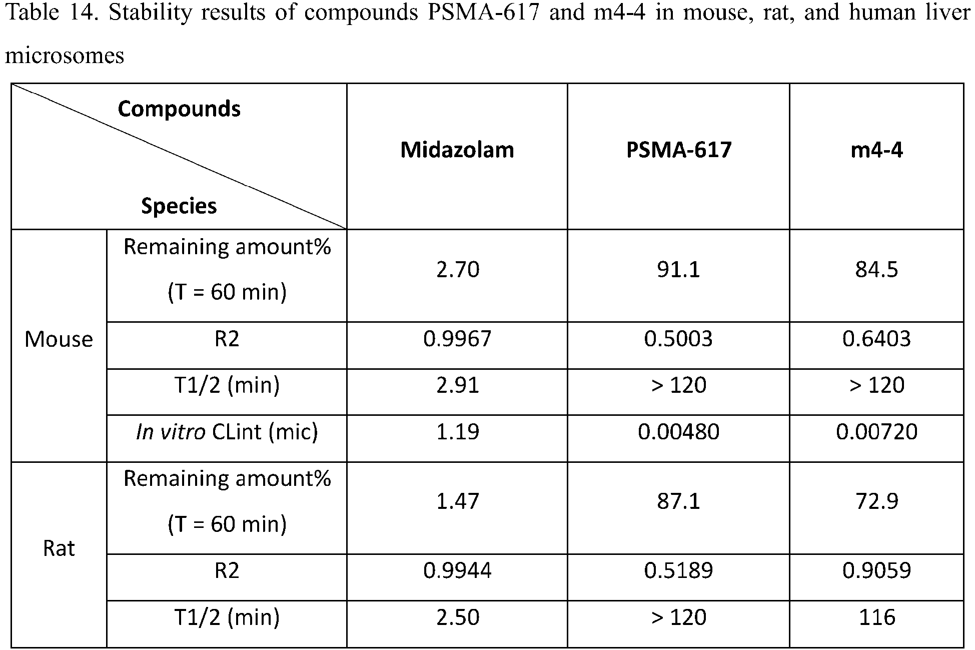

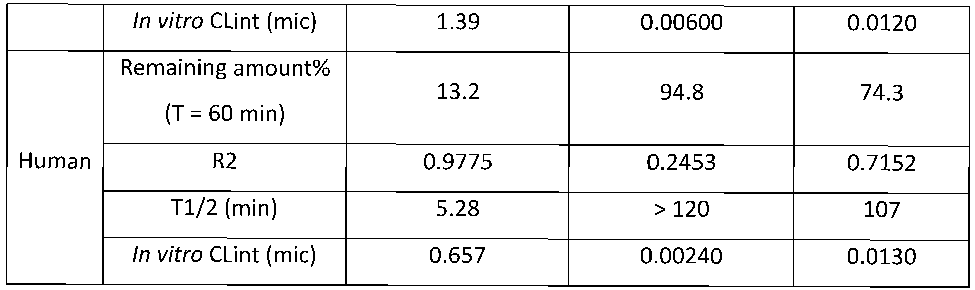

**Table 15. Metabolic stability results of compounds PSMA-617 and m4-4 conjugated with nuclide ¹⁷⁵Lu in mouse, rat, and human liver microsomes**

| Compound | Species | Test concentration (µM) | +/cofactor | Remaining amount (%)^{§} | | | | | t1/2 (min) | CLint (µL/min /mg protein) |
|---|---|---|---|---|---|---|---|---|---|---|
| | | | | 0.5 min | 15 min | 30 min | 45 min | 60 min | | |
| Verapamil | Human | 2 | + | 100 .00 | 17. 53 | 7.4 1 | 4.13 | 2.76 | 11.9 9 | 115.57 |
| | | | - | 100 .00 | 101 .17 | 97. 43 | 97.6 3 | 101. 74 | | |
| Verapamil | Rat | 2 | + | 100 .00 | 6.4 3 | 1.3 3 | 0.49 | 0.22 | 4.74 | 292.39 |
| | | | - | 100 .00 | 99. 09 | 91. 23 | 93.6 9 | 101. 07 | | |
| Verapamil | Mouse | 2 | + | 100 .00 | 7.8 2 | 2.0 7 | 0.72 | 0.32 | 5.28 | 262.37 |
| | | | | 100 .00 | 92. 47 | 93. 05 | 94.3 3 | 89.2 6 | | |
| ¹⁷⁵Lu-PSMA -617 | Human | 2 | + | 100 .00 | 94. 31 | 95. 87 | 94.4 0 | 106. 52 | > 184. 78* | < 7.50^{*} |
| | | | - | 100 .00 | 99. 41 | 116 .80 | 106. 74 | 110. 50 | | |
| ¹⁷⁵Lu-PSMA -617 | Rat | 2 | + | 100 .00 | 104 .37 | 98. 24 | 98.9 9 | 94.0 0 | > 184. 78* | < 7.50^{*} |
| | | | - | 100 .00 | 102 .80 | 107 .48 | 105. 79 | 92.5 0 | | |
| ¹⁷⁵Lu-PSMA-617 | Mouse | 2 | + | 100 .00 | 95. 94 | 97. 59 | 89.7 5 | 102. 13 | > 184. | < 7.50^{*} |
| | | | | 100 .00 | 87. 37 | 90. 92 | 98.5 7 | 97.3 1 | 78* | |
| ¹⁷⁵Lu-m4-4 | Human | 2 | + | 100 .00 | 86. 52 | 90. 54 | 98.1 8 | 86.3 8 | > 184. 78* | < 7.50^{*} |
| | | | - | 100 .00 | 87. 30 | 87. 90 | 89.5 6 | 95.8 8 | | |
| ¹⁷⁵Lu-m4-4 | Rat | 2 | + | 100 .00 | 88. 89 | 97. 60 | 94.1 6 | 97.2 2 | > 184. 78* | < 7.50^{*} |
| | | | - | 100 .00 | 94. 41 | 95. 52 | 91.5 1 | 96.5 6 | | |
| ¹⁷⁵Lu-m4-4 | Mouse | 2 | + | 100 .00 | 97. 18 | 95. 73 | 93.3 5 | 104. 08 | > 184. 78* | < 7.50^{*} |
| | | | - | 100 .00 | 82. 30 | 98. 30 | 96.2 2 | 92.9 4 | | |

| | | | | | | | | | | |
|---|---|---|---|---|---|---|---|---|---|---|
| Symbol § represents that: remaining amounts (%) of < 1% were not used for t_{1/2} and CLint calculation. Symbol * represents that: t1/2 is reported as > 184.78 and CLint is reported as < 7.50 when the remaining amount (%) of the compound at 60 min is > 80%. | | | | | | | | | | |

The results of liver microsomal stability assay are as follows:
PSMA-617 > (mouse) 120 min, 0.00480 mic or > (rat) 120 min, 0.00600 mic or > (human) 120 min, 0.00240 mic;
m4-4 > (mouse) 120 min, 0.00720 mic or (rat) 116 min, 0.0120 mic or (human) 107 min, 0.0130 mic.

By comparing stability results of the control compound PSMA-617 and m4-4 in the liver microsome, the clearance rates of PSMA-617 and m4-4 in liver microsomal stability experiments in mice, rats, and humans are comparable, at the same level. Further investigation was conducted to determine whether the clearance rate changed after the compounds chelated the heavy metal ¹⁷⁵Lu. The results show that:
both ¹⁷⁵Lu-PSMA-617 and ¹⁷⁵Lu-m4-4 exhibit low clearance rates in mouse, rat, and human liver microsomal stability assays, specifically: t1/2 > 184.78 min, CLint < 7.5 µL/min/mg. These compounds were subjected to subsequent *in vivo* experiments to evaluate their respective plasma drug concentration.

### 8. In vivo pharmacokinetics (PK)

The pharmacokinetics of ¹⁷⁵Lu-PSMA-617 and ¹⁷⁵Lu-m4-4 in rats were studied. Results are shown in Tables 16-17.

### 8.1 Experimental materials:

### Animal lines

| Species | Line, gender | Grade | Age of animals | Weight |
|---|---|---|---|---|
| Rat | SD rat, male | SPF | 6-8 weeks | 180-220 g |

### Reagent information

| Reagent name | CAS. No. | Cat. No. | Source |
|---|---|---|---|
| DMSO | 67-68-5 | A00878 | Sigma-Aldrich |
| Solutol HS | 70142-34-6 | P916874 | Macklin |
| Saline | 7647-14-5 | 2203091905 | Shimen |

### 8.2 Experimental method:

| Compound name | Route of administratio n | Dose of administratio n | Solvent | Number of animals | Fasting or not |
|---|---|---|---|---|---|
| ¹⁷⁵Lu-PSMA-617 | IV | 2 mpk | 5% DMSO+10% Solutol+85% Saline | N = 3 | NO |
| ¹⁷⁵Lu-m4-4 | IV | 2 mpk | 5% DMSO+10% Solutol+85% Saline | N = 3 | NO |

Blood samples were collected from the jugular vein at 5 min, 15 min, 30 min, 1 h, 2 h, 4 h, 7 h, and 24 h after IV (intravenous) administration. The blood volume collected each time was 0.2 mL. The collected blood samples were transferred to microcentrifuge tubes containing EDTA-K2 anticoagulant, centrifuged at 4°C and 4000 g for 5 min, and the supernatant was collected and stored in a refrigerator at -75°C ± 15°C. Protein precipitation was adopted for sample pretreatment, and LC-MS/MS was adopted to measure and analyze the concentrations of the plasma samples. The obtained data was used to calculate the pharmacokinetic parameters using WinNonlin software. The pharmacokinetic parameters regarding IV administration include CL, t1/2, C0, AUC, MRT, Vd, and so on.

### 8.3 Data analysis:

**Table 16. Plasma drug concentration and PK parameters of ¹⁷⁵Lu-PSMA-617 after intravenous injection of 2 mg/kg ¹⁷⁵Lu-PSMA-617 in SD rats**

| **Dose (mg/kg)** | **Route of administration** | **Blood collection point** | **Concentration (ng/mL) (1)** | | | **Average (ng/mL)** | **Standard deviation** | **Coefficient of variation (%)** |
|---|---|---|---|---|---|---|---|---|
| | | | **Gender/ M** | **Gender/ M** | **Gender/ M** | | | |
| | | | **101-M** | **102-M** | **103-M** | | | |
| 2 | IV | 5 min | 5550 | 5260 | 4920 | 5243 | 315 | 6.01 |
| | | 15 min | 3630 | 3550 | 3320 | 3500 | 161 | 4.60 |
| | | 30 min | 2040 | 2830 | 2590 | 2487 | 405 | 16.3 |
| | | 1 h | 1150 | 1430 | 1250 | 1277 | 142 | 11.1 |
| | | 2 h | 306 | 264 | 236 | 269 | 35.2 | 13.1 |
| | | 4 h | 17.0 | BQL | BQL | NA | NA | NA |
| | | 8 h | BQL | BQL | BQL | NA | NA | NA |
| | | 24 h | BQL | BQL | BQL | NA | NA | NA |

| PK paramet ers | WinNonlin Parameter | Unit | 101 | 102 | 103 | Average | Standard deviation | Coefficient of variation (%) |
|---|---|---|---|---|---|---|---|---|
| t1/2 | HL_Lambda_z | h | 0.491 | 0.434 | 0.431 | 0.452 | 0.0339 | 7.50 |
| Kel | Lambda_z | 1/h | 1.41 | 1.60 | 1.61 | 1.54 | 0.111 | 7.19 |
| Vdss | Vss_obs | L/kg | 0.342 | 0.303 | 0.326 | 0.324 | 0.0195 | 6.01 |
| MRTinf | MRTINF_obs | h | 0.658 | 0.621 | 0.608 | 0.629 | 0.0260 | 4.13 |
| Cl | Cl_obs | mL/min/k g | 8.65 | 8.14 | 8.94 | 8.58 | 0.404 | 4.71 |
| AUC0-t | AUClast | h*ng/mL | 3839 | 3930 | 3583 | 3784 | 180 | 4.75 |
| AUC0-inf | AUCINF_obs | h*ng/mL | 3851 | 4095 | 3730 | 3892 | 186 | 4.78 |
| AUC0-t/AUC0-inf | | / | 0.997 | 0.960 | 0.961 | 0.972 | 0.0212 | 2.18 |

| | | | | | | | | |
|---|---|---|---|---|---|---|---|---|
| BQL in columns with symbol (1) represents below the lower limit of quantification measurement (SD rat plasma 10.0 ng/mL) | | | | | | | | |

**Table 17. Plasma drug concentration and PK parameters of ¹⁷⁵Lu-m4-4 after intravenous injection of 2 mg/kg ¹⁷⁵Lu-m4-4 in SD rats**

| Dose (mg/kg) | Route of administration | Blood collectio n point | Concentration (ng/mL) (1) | | | Average (ng/mL) | Standard deviation | Coefficient of variation (%) |
|---|---|---|---|---|---|---|---|---|
| | | | Gender/ M | Gender/M | Gender /M | | | |
| | | | 301-M | 302-M | 303-M | | | |
| 2 | IV | 5 min | 9480 | 10300 | 8940 | 9573 | 685 | 7.15 |
| | | 15 min | 3590 | 5250 | 3860 | 4233 | 891 | 21.0 |
| | | 30 min | 2590 | 2810 | 2790 | 2730 | 122 | 4.46 |
| | | 1 h | 1140 | 2020 | 1040 | 1400 | 539 | 38.5 |
| | | 2 h | 216 | 505 | 167 | 296 | 183 | 61.7 |
| | | 4 h | 18.4 | 15.9 | BQL | 17.2 | NA | NA |
| | | 8 h | BQL | BQL | BQL | NA | NA | NA |
| | | 24 h | BQL | BQL | BQL | NA | NA | NA |

| PK parameters | WinNonlin Parameter | Unit | 201 | 202 | 203 | Average | Standard deviation | Coefficient of variation (%) |
|---|---|---|---|---|---|---|---|---|
| tl/2 | HL_Lambda_ z | h | 0.488 | 0.425 | 0.371 | 0.428 | 0.0587 | 13.7 |
| Kel | Lambda_z | 1/h | 1.42 | 1.63 | 1.87 | 1.64 | 0.225 | 13.7 |
| Vdss | Vss_obs | L/kg | 0.213 | 0.202 | 0.198 | 0.204 | 0.00792 | 3.88 |
| MRTinf | MRTINF_obs | h | 0.506 | 0.639 | 0.444 | 0.530 | 0.0996 | 18.8 |
| Cl | Cl_obs | mL/min/ kg | 7.01 | 5.26 | 7.43 | 6.57 | 1.15 | 17.5 |
| AUC0-t | AUClast | h*ng/m L | 4743 | 6324 | 4398 | 5155 | 1027 | 19.9 |
| AUC0-inf | AUCINF_obs | h*ng/m L | 4756 | 6334 | 4487 | 5192 | 998 | 19.2 |
| AUC0-t/AUC0-inf | | / | 0.997 | 0.998 | 0.980 | 0.992 | 0.0103 | 1.04 |

| | | | | | | | | |
|---|---|---|---|---|---|---|---|---|
| BQL in columns with symbol (1) represents below the lower limit of quantification measurement (SD rat plasma 10.0 ng/mL) | | | | | | | | |

Results *In vivo* pharmacokinetic (PK) experiments of the cold compounds ¹⁷⁵Lu-PSMA-617 and ¹⁷⁵Lu-m4-4 show that the t_{1/2} of ¹⁷⁵Lu-PSMA-617 and ¹⁷⁵Lu-m4-4 are 0.452 h and 0.428 h, respectively, and their AUC₀₋ₜ are 3784 h*ng/mL and 5155 h*ng/mL, respectively. This suggests that the *in vivo* clearance rate of the compound ¹⁷⁵Lu-m4-4 of the present disclosure is at the same level as that of the control compound ¹⁷⁵Lu-PSMA-617, but the AUC_{0-inf} and AUC₀₋ₜ *in vivo* of ¹⁷⁵Lu-m4-4 are significantly higher, with the total exposure *in vivo* higher than that of ¹⁷⁵Lu-PSMA-617. Therefore, the compound of the present disclosure facilitates accumulation in tumor tissue.

### Example 4 Radiolabeled compound experiment

Experiments were conducted on the radiolabeled compounds ¹⁷⁷Lu-PSMA-617 and ¹⁷⁷Lu-m4-4

### 1.1 Experimental animal

Animal strain: NCG mouse
Animal grade: SPF grade
Animal gender: Female
Animal age: 9-10 w
Animal weight: 18-22 g
Animal source: Beijing GemPharmatech Co., Ltd., China

### 1.2 Experimental design

### 1.2.1 Dose and grouping

The animals were randomly divided to groups based on tumor volume and body weight. The compounds were administered at 50 µCi per mouse.

### 1.2.2 Route of administration

Route of administration: Intravenous injection via the tail vein
Administration frequency: Single dose

### 1.3 Analysis and detection

Sampling time: 0.5 hours, 1 hour, 4 hours and 8 hours post-administration.

Tissue and organ: Blood was collected via retro-orbital bleeding (blood samples were retained). The following organs/tissues were excised: brain, heart, lungs, liver, stomach (contents removed), salivary glands, prostate, small intestine (contents removed), large intestine (contents removed), spleen, pancreas, kidneys (without adrenal gland), bladder, femur (hind limb), muscle, testis, tumor, and residual tissue.

Gamma counter detection: Biological samples collected at each time point were gently blotted with absorbent paper to remove residual blood, weighed, and recorded. The samples were placed in EP tubes and detected using a gamma counter. The detection results are expressed as CPM (CPM: Radiation Counts Per Minute). If the radiation counts were too high, the samples were allowed to be left for decay before measurement.

### 1.4 Experimental results

Tables 18-19 show the %ID/g of ¹⁷⁷Lu-PSMA-617 and ¹⁷⁷Lu-m4-4 at different time points within 0-8 h post-administration:

**Table 18. %ID/g of ¹⁷⁷Lu-PSMA-617 at different time points**

| | Sampling time | %ID/g | | | | | |
|---|---|---|---|---|---|---|---|
| | | Blood | Brain | Heart | Lungs | Liver | Stomach |
| ¹⁷⁷Lu-PSMA-617 | 0.5 h | 2.91 | 0.11 | 0.99 | 2.74 | 0.61 | 1.83 |
| | | 1.23 | 0.08 | 0.57 | 1.44 | 0.44 | 0.98 |
| | | 2.08 | 0.16 | 1.11 | 2.59 | 0.63 | 1.26 |
| | Average | 2.07 | 0.12 | 0.89 | 2.26 | 0.56 | 1.36 |
| | SD | 0.84 | 0.04 | 0.29 | 0.71 | 0.10 | 0.43 |
| ¹⁷⁷Lu-PSMA-617 | 1 h | 0.50 | 0.23 | 0.39 | 1.16 | 0.45 | 0.84 |
| | | 0.67 | 0.06 | 0.38 | 1.11 | 0.35 | 0.80 |
| | | 0.50 | 0.14 | 0.28 | 0.82 | 0.25 | 0.48 |
| | Average | 0.55 | 0.15 | 0.35 | 1.03 | 0.35 | 0.70 |
| | SD | 0.10 | 0.08 | 0.06 | 0.19 | 0.10 | 0.20 |
| ¹⁷⁷Lu-PSMA-617 | 4 h | 0.04 | 0.06 | 0.07 | 0.32 | 0.14 | 0.15 |
| | | 0.08 | 0.02 | 0.12 | 0.29 | 0.34 | 0.22 |
| | | 0.05 | 0.02 | 0.06 | 0.15 | 0.11 | 0.88 |
| | Average | 0.06 | 0.03 | 0.08 | 0.25 | 0.20 | 0.42 |
| | SD | 0.02 | 0.02 | 0.04 | 0.09 | 0.13 | 0.40 |
| ¹⁷⁷Lu-PSMA-617 | 8 h | 0.04 | 0.04 | 0.06 | 0.18 | 0.11 | 0.44 |
| | | 0.08 | 0.03 | 0.05 | 0.13 | 0.11 | 0.14 |
| | | 0.06 | 0.02 | 0.13 | 0.35 | 0.08 | 0.24 |
| | Average | 0.06 | 0.03 | 0.08 | 0.22 | 0.10 | 0.27 |
| | SD | 0.02 | 0.01 | 0.05 | 0.12 | 0.01 | 0.15 |

| | Sampling time | %ID/g | | | | | |
|---|---|---|---|---|---|---|---|
| | | Small intestin e | Large intestine | Salivary gland | Spleen | Pancreas | Kidneys |
| ¹⁷⁷Lu-PSMA-617 | 0.5 h | 1.43 | 1.60 | 1.47 | 5.22 | 0.87 | 148.76 |
| | | 0.76 | 0.82 | 0.89 | 2.44 | 0.49 | 52.69 |
| | | 1.20 | 1.61 | 1.33 | 5.66 | 1.09 | 163.52 |
| | Average | 1.13 | 1.34 | 1.23 | 4.44 | 0.82 | 121.66 |
| | SD | 0.34 | 0.45 | 0.30 | 1.75 | 0.30 | 60.18 |
| ¹⁷⁷Lu-PSMA-617 | 1 h | 0.49 | 0.88 | 0.44 | 2.17 | 0.47 | 39.49 |
| | | 0.41 | 1.34 | 0.61 | 2.24 | 0.33 | 61.14 |
| | | 1.02 | 0.82 | 0.45 | 2.49 | 0.29 | 35.25 |
| | Average | 0.64 | 1.01 | 0.50 | 2.30 | 0.37 | 45.29 |
| | SD | 0.33 | 0.29 | 0.09 | 0.17 | 0.10 | 13.88 |
| ¹⁷⁷Lu-PSMA-617 | 4 h | 0.18 | 0.27 | 0.12 | 0.29 | 0.05 | 5.74 |
| | | 0.12 | 1.00 | 0.16 | 0.79 | 0.09 | 13.91 |
| | | 0.20 | 0.35 | 0.11 | 1.22 | 0.07 | 4.43 |
| | Average | 0.17 | 0.54 | 0.13 | 0.77 | 0.07 | 8.03 |
| | SD | 0.04 | 0.40 | 0.03 | 0.47 | 0.02 | 5.14 |
| ¹⁷⁷Lu-PSMA-617 | 8 h | 0.09 | 0.29 | 0.10 | 0.31 | 0.19 | 3.82 |
| | | 0.11 | 0.69 | 0.09 | 0.20 | 0.06 | 3.96 |
| | | 0.11 | 0.17 | 0.08 | 0.46 | 0.04 | 4.13 |
| | Average | 0.10 | 0.38 | 0.09 | 0.32 | 0.10 | 3.97 |
| | SD | 0.01 | 0.27 | 0.01 | 0.13 | 0.08 | 0.16 |

| Bladder | Femur | Muscle | Prostate | Testis | Tumor | | |
|---|---|---|---|---|---|---|---|
| ¹⁷⁷Lu-PSMA-617 | 0.5 h | 16.69 | 1.30 | 0.64 | 3.59 | 2.01 | 38.71 |
| | | 19.98 | 0.88 | 0.72 | 8.07 | 1.45 | 41.65 |
| | | 4.89 | 0.83 | 0.50 | 2.40 | 0.80 | 52.28 |
| | Average | 13.85 | 1.00 | 0.62 | 4.69 | 1.42 | 44.22 |
| | SD | 7.93 | 0.26 | 0.11 | 2.99 | 0.61 | 7.14 |
| ¹⁷⁷Lu-PSMA-617 | 1 h | 8.86 | 0.57 | 0.26 | 9.59 | 0.53 | 35.31 |
| | | 26.93 | 1.59 | 1.19 | 9.31 | 1.68 | 40.32 |
| | | 14.00 | 0.37 | 0.19 | 3.85 | 0.25 | 49.74 |
| | Average | 16.60 | 0.84 | 0.55 | 7.58 | 0.82 | 41.79 |
| | SD | 9.31 | 0.65 | 0.56 | 3.23 | 0.76 | 7.32 |
| ¹⁷⁷Lu-PSMA-617 | 4 h | 0.62 | 0.22 | 0.12 | 0.97 | 0.09 | 42.80 |
| | | 21.99 | 0.47 | 0.53 | 10.54 | 6.08 | 48.93 |
| | | 1.06 | 0.43 | 0.08 | 0.42 | 0.19 | 31.19 |
| | Average | 7.89 | 0.37 | 0.24 | 3.98 | 2.12 | 40.97 |
| | SD | 12.21 | 0.13 | 0.25 | 5.69 | 3.43 | 9.01 |
| ¹⁷⁷Lu-PSMA-617 | 8 h | 1.59 | 0.35 | 0.11 | 0.91 | 0.20 | 51.91 |
| | | 0.30 | 0.21 | 0.04 | 0.36 | 0.09 | 40.66 |
| | | 0.31 | 0.25 | 0.03 | 0.08 | 0.05 | 40.77 |
| | Average | 0.73 | 0.27 | 0.06 | 0.45 | 0.11 | 44.45 |
| | SD | 0.74 | 0.07 | 0.05 | 0.42 | 0.08 | 6.46 |

**Table 19. %ID/g of ¹⁷⁷Lu-m4-4 at different time points**

| | Sampling time | %ID/g | | | | | |
|---|---|---|---|---|---|---|---|
| | | Blood | Brain | Heart | Lungs | Liver | Stomach |
| ¹⁷⁷Lu-m4-4 | 0.5 h | 3.01 | 0.11 | 1.69 | 3.71 | 0.81 | 2.14 |
| | | 2.11 | 0.09 | 1.57 | 2.63 | 0.64 | 1.65 |
| | | 1.99 | 0.07 | 1.32 | 1.98 | 0.50 | 1.62 |
| | | 1.94 | 0.09 | 1.02 | 2.47 | 0.50 | 1.15 |
| | | 3.12 | 0.14 | 1.85 | 3.35 | 0.90 | 2.40 |
| | Average | 2.35 | 0.10 | 1.40 | 2.60 | 0.63 | 1.73 |
| ¹⁷⁷Lu-m4-4 | 1 h | 0.61 | 0.05 | 0.91 | 1.48 | 0.42 | 0.93 |
| | | 1.20 | 0.06 | 0.82 | 2.42 | 0.82 | 1.61 |
| | | 0.65 | 0.05 | 0.54 | 1.93 | 0.35 | 0.81 |
| | | 0.58 | 0.08 | 0.65 | 4.97 | 0.49 | 1.05 |
| | | 0.55 | 0.04 | 0.61 | 1.38 | 0.31 | 0.74 |
| | Average | 0.59 | 0.06 | 0.60 | 2.76 | 0.38 | 0.87 |
| ¹⁷⁷Lu-m4-4 | 4 h | 0.08 | 0.02 | 0.25 | 0.35 | 0.14 | 0.22 |
| | | 0.11 | 0.04 | 0.22 | 0.47 | 0.25 | 1.51 |
| | | 0.13 | 0.02 | 0.20 | 0.38 | 0.20 | 0.39 |
| | | 0.18 | 0.03 | 0.58 | 0.56 | 0.32 | 0.32 |
| | | 0.06 | 0.06 | 0.13 | 0.33 | 0.17 | 0.30 |
| | Average | 0.12 | 0.04 | 0.30 | 0.42 | 0.23 | 0.34 |
| ¹⁷⁷Lu-m4-4 | 8 h | 0.06 | 0.03 | 0.07 | 0.12 | 0.09 | 0.10 |
| | | 0.03 | 0.03 | 0.07 | 0.14 | 0.11 | 0.15 |
| | | 0.05 | 0.03 | 0.08 | 3.08 | 0.12 | 0.19 |
| | | 0.04 | 0.02 | 0.08 | 0.15 | 0.11 | 0.16 |
| | | 0.04 | 0.03 | 0.11 | 0.11 | 0.12 | 0.19 |
| | Average | 0.04 | 0.03 | 0.09 | 1.11 | 0.12 | 0.18 |

| | Sampling time | %ID/g | | | | | |
|---|---|---|---|---|---|---|---|
| | | Small intestine | Large intestine | Salivary gland | Spleen | Pancreas | Kidneys |
| ¹⁷⁷Lu-m4-4 | 0.5 h | 1.84 | 1.72 | 2.08 | 8.64 | 1.00 | 134.67 |
| | | 1.18 | 1.62 | 2.16 | 17.05 | 1.10 | 155.37 |
| | | 1.92 | 1.57 | 1.66 | 10.34 | 0.92 | 151.67 |
| | | 1.46 | 1.26 | 1.47 | 8.80 | 1.10 | 134.53 |
| | | 2.21 | 2.34 | 2.38 | 10.74 | 0.79 | 102.68 |
| | Average | 1.87 | 1.72 | 1.84 | 9.96 | 0.94 | 129.63 |
| ¹⁷⁷Lu-m4-4 | 1 h | 0.60 | 0.88 | 0.98 | 10.23 | 0.68 | 168.88 |
| | | 1.07 | 1.26 | 1.83 | 15.81 | 1.08 | 173.08 |
| | | 0.96 | 0.68 | 1.08 | 11.80 | 0.64 | 198.47 |
| | | 0.77 | 0.73 | 1.22 | 13.55 | 0.92 | 191.49 |
| | | 1.19 | 1.75 | 1.19 | 6.04 | 0.64 | 184.90 |
| | Average | 0.97 | 1.06 | 1.16 | 10.47 | 0.74 | 191.62 |
| ¹⁷⁷Lu-m4-4 | 4 h | 0.24 | 0.31 | 0.28 | 1.06 | 0.14 | 74.36 |
| | | 0.23 | 0.50 | 0.40 | 2.91 | 0.31 | 95.10 |
| | | 0.38 | 0.63 | 0.37 | 4.33 | 0.21 | 89.81 |
| | | 0.36 | 0.55 | 0.43 | 4.20 | 0.28 | 75.02 |
| | | 0.30 | 0.33 | 0.22 | 1.31 | 0.17 | 73.50 |
| | Average | 0.35 | 0.50 | 0.34 | 3.28 | 0.22 | 79.44 |
| ¹⁷⁷Lu-m4-4 | 8 h | 0.15 | 0.12 | 0.16 | 0.52 | 0.07 | 19.93 |
| | | 0.09 | 0.13 | 0.15 | 0.35 | 0.07 | 16.85 |
| | | 0.23 | 0.68 | 0.21 | 1.65 | 0.16 | 10.21 |
| | | 0.12 | 0.22 | 0.20 | 0.56 | 0.09 | 12.80 |
| | | 0.10 | 0.16 | 0.19 | 0.97 | 0.14 | 17.29 |
| | Average | 0.15 | 0.35 | 0.20 | 1.06 | 0.13 | 13.43 |

| | Sampling time | %ID/g | | | | | |
|---|---|---|---|---|---|---|---|
| | | Bladder | Femur | Muscle | Prostate | Testis | Tumor |
| ¹⁷⁷Lu-m4-4 | 0.5 h | 3.68 | 1.24 | 0.71 | 4.17 | 0.86 | 63.73 |
| | | 9.15 | 0.98 | 0.69 | 1.85 | 0.97 | 75.58 |
| | | 4.74 | 0.89 | 0.46 | 1.35 | 0.87 | 78.63 |
| | | 6.01 | 0.95 | 0.69 | 2.89 | 0.84 | 63.84 |
| | | 5.69 | 1.34 | 0.84 | 1.49 | 0.74 | 69.03 |
| | Average | 5.48 | 1.06 | 0.66 | 1.91 | 0.81 | 70.50 |
| ¹⁷⁷Lu-m4-4 | 1 h | 0.25 | 0.39 | 0.25 | 1.05 | 0.70 | 60.14 |
| | | 6.73 | 0.71 | 0.42 | 2.05 | 0.90 | 57.00 |
| | | 1.64 | 0.46 | 0.27 | 0.87 | 0.72 | 68.75 |
| | | 3.60 | 0.47 | 0.34 | 1.69 | 1.10 | 59.16 |
| | | 5.03 | 0.62 | 0.28 | 3.72 | 0.81 | 76.50 |
| | Average | 3.42 | 0.52 | 0.29 | 2.10 | 0.88 | 68.14 |
| ¹⁷⁷Lu-m4-4 | 4 h | 7.46 | 0.35 | 0.14 | 2.26 | 0.73 | 70.23 |
| | | 5.08 | 0.28 | 0.16 | 1.29 | 0.30 | 73.81 |
| | | 0.98 | 0.18 | 0.13 | 0.52 | 0.29 | 65.63 |
| | | 2.42 | 0.99 | 0.34 | 18.09 | 0.73 | 68.85 |
| | | 2.21 | 0.25 | 0.14 | 9.74 | 0.50 | 76.68 |
| | Average | 1.87 | 0.47 | 0.20 | 9.45 | 0.51 | 70.39 |
| ¹⁷⁷Lu-m4-4 | 8 h | 14.60 | 0.10 | 0.05 | 0.58 | 0.19 | 71.62 |
| | | 6.81 | 0.54 | 0.12 | 2.08 | 0.16 | 73.77 |
| | | 0.94 | 0.12 | 0.04 | 1.20 | 0.20 | 75.47 |
| | | 1.95 | 0.12 | 0.04 | 2.34 | 0.13 | 66.06 |
| | | 6.69 | 0.45 | 0.20 | 1.49 | 0.19 | 74.59 |
| | Average | 3.20 | 0.23 | 0.10 | 1.68 | 0.18 | 72.04 |

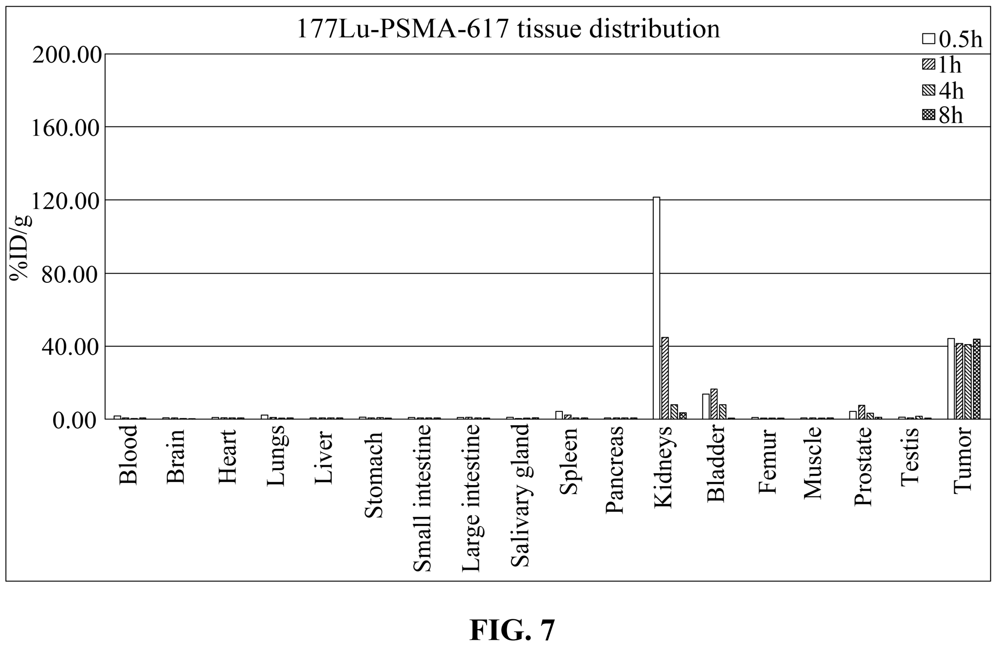

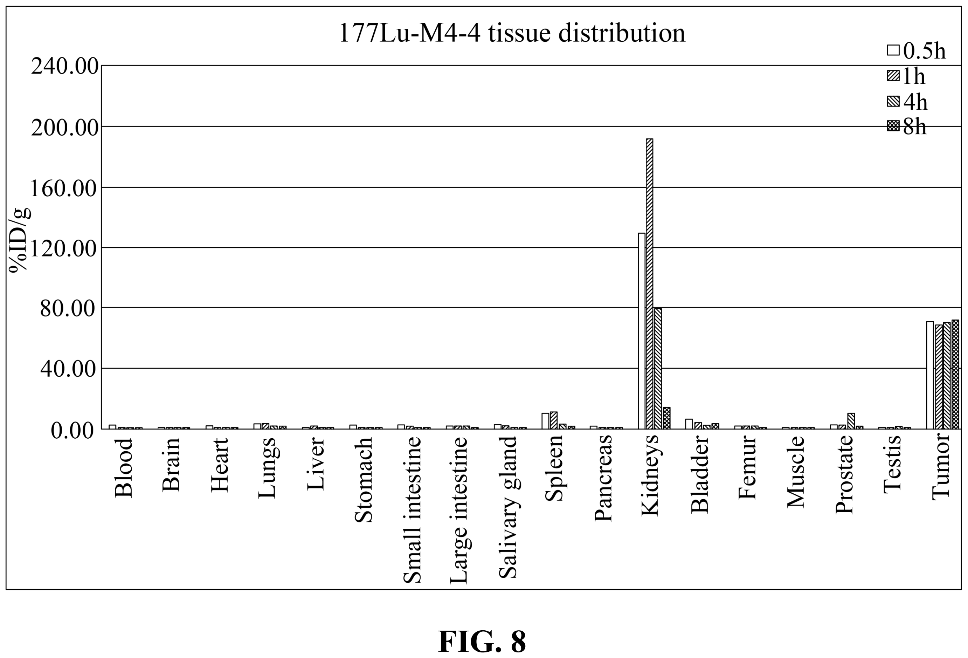

The tissue distribution results of the radiolabeled compounds are shown in FIG. 7 to FIG. 8. Both compounds are absorbed and distributed to a given extent in the spleen, kidneys, bladder, prostate, and tumor, with the largest absorption amount observed in the tumor tissues and kidneys. Compared with ¹⁷⁷Lu-PSMA617, ¹⁷⁷Lu-m4-4 exhibits significantly greater accumulation effect at the tumor site, suggesting superior tumor inhibition effects. In addition, compound m4-4 exhibits higher kidney exposure than compound PSMA-617 before 4 h. However, at 8 h, the exposure levels of both compounds in the kidneys are comparable, and no major safety concerns are noted.

The above descriptions are merely several exemplary embodiments of the present disclosure and are not intended to limit the present disclosure in any form. Although the present disclosure has been disclosed as above by way of preferred embodiments, it is not intended to limit the present disclosure. Any equivalent or similar embodiments obtained by those skilled in the art by making some changes or modifications to the above-disclosed technical content without departing from the scope of technical solutions of the present disclosure are within the scope of the present disclosure.

## Claims

1. A prostate-specific membrane antigen (PSMA)-targeting ligand compound or a stereoisomer, a tautomer, a hydrate, a solvate, a pharmaceutically acceptable salt or ester thereof, wherein the PSMA-targeting ligand compound has a structure as shown in formula I, 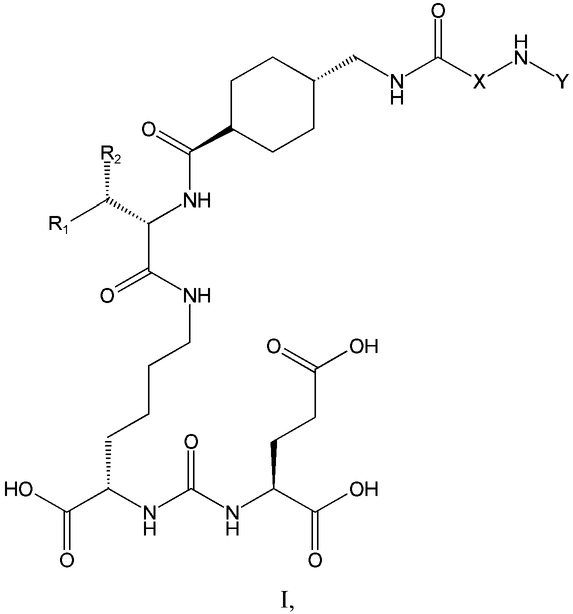
wherein R₁ is selected from the group consisting of optionally substituted aryl and optionally substituted heteroaryl, a substituent in the optionally substituted aryl or the optionally substituted heteroaryl is selected from the group consisting of: deuterium, carboxyl, halogen, linear or branched C1-C5 alkyl substituted with halogen, linear or branched C1-C5 alkylacyl, linear or branched C1-C5 alkyl ester group, hydroxyl, linear or branched C1-C5 alkylthio, cyano, linear or branched C1-C5 alkoxy, amino, nitro, phenylsulfonamido, linear or branched C1-C5 alkylamido, linear or branched C2-C6 alkenyl, and linear or branched C2-C6 alkynyl;
R₂ is selected from the group consisting of: hydrogen, deuterium, carboxyl, halogen, unsubstituted linear or branched C1-C5 alkyl, linear or branched C1-C5 alkyl substituted with halogen, linear or branched C1-C5 alkylacyl, linear or branched C1-C5 alkyl ester group, hydroxyl, linear or branched C1-C5 alkylthio, cyano, linear or branched C1-C5 alkoxy, amino, nitro, phenylsulfonamido, linear or branched C1-C5 alkylamido, linear or branched C2-C6 alkenyl, and linear or branched C2-C6 alkynyl;
X is -Z-(CH₂)ₙ-(CR₃R₄)-, wherein Z is a chemical bond, -NH-, or -(CH₂)-; n is 0-5; and R₃ and R₄ are each independently selected from the group consisting of: hydrogen, carboxyl, and optionally substituted linear or branched C1-C5 alkyl, a substituent in the optionally substituted linear or branched C1-C5 alkyl is selected from the group consisting of: halogen, hydroxyl, amino, carboxyl, amido, -CN, -CF₃, nitro, sulfonyl, linear or branched C1-C5 alkoxy, linear or branched C1-C5 alkylamino, linear or branched C2-C6 alkenyl, linear or branched C2-C6 alkynyl, five-membered or six-membered aryl, linear or branched C1-C5 heteroalkyl, five-membered or six-membered heteroaryl, five-membered or six-membered heterocycloalkyl, linear or branched C1-C5 alkyl substituted with five-membered or six-membered aryl, five-membered or six-membered aryl substituted with linear or branched C1-C5 alkyl, five-membered or six-membered heteroaryl substituted with linear or branched C1-C5 alkyl, linear or branched C1-C5 alkyl substituted with five-membered or six-membered heteroaryl, and biphenyl; and
Y is a radioactive metal ion chelating group.

2. The PSMA-targeting ligand compound or the stereoisomer, the tautomer, the hydrate, the solvate, the pharmaceutically acceptable salt or ester thereof as claimed in claim 1, wherein
under a condition that Z is the -NH-, both the R₃ and the R₄ are hydrogen;
under a condition that Z is the -(CH₂)-, one of the R₃ and the R₄ is hydrogen, and an other is carboxyl; or
under a condition that Z is the chemical bond, n is 0, one of the R₃ and the R₄ is hydrogen, and an other is linear or branched C1-C5 alkyl substituted with carboxyl.

3. The PSMA-targeting ligand compound or the stereoisomer, the tautomer, the hydrate, the solvate, the pharmaceutically acceptable salt or ester thereof as claimed in claim 1, wherein
R₁ is selected from the group consisting of optionally substituted phenyl and optionally substituted naphthyl, wherein a substituent in the optionally substituted phenyl or the optionally substituted naphthyl is selected from the group consisting of halogen, such as F-, Cl-, Br- or I-, cyano, trifluoromethyl, difluoromethyl, and monofluoromethyl;
preferably, R₂ is selected from the group consisting of hydrogen and unsubstituted linear or branched C1-C5 alkyl;
preferably, X is selected from the group consisting of -NH-(CH₂)-, -(CH₂)-(CH₂)-(CHCOOH)-, -(CH-CH₂-CH₂COOH)-, -(CH-CH₂-COOH)-, -NH-(CH₂)-(CH₂)-, -(CH₂)-(CHCOOH)-, and -(CH₂)-(CH₂)-(CH₂)-(CHCOOH)-;
preferably, Y is the radioactive metal ion chelating group formed from a chelating agent selected from the group consisting of: 1,4,7,10-tetraazacyclododecane-1,4,7,10-tetraacetic acid, 1,4,7-triazacyclononane-1,4,7-triacetic acid, 1,4,7-triazacyclononane triacetic acid modified with an amino acid, diethylenetriamine pentaacetic acid, 1,4,7-triazacyclononane-1,4-diacetic acid, 2-(4,7-bis(carboxymethyl)-1,4,7-triazonon-1-yl)glutaric acid, 1,4,7,10-tetraazacyclododecane-1,4,7,10-tetramethylene phosphonic acid, 1,4,8,11-tetraazacyclotetradecane-1,4,8,11-tetraacetic acid, diacetylbis(N4-methyl-3-thiosemicarbazone), pyruvaldehyde bis(N4,N4-dimethylthiosemicarbazone), ethylenediamine tetraacetic acid, trisodium ethylenediamine disuccinate, N,N'-bis(2-hydroxybenzyl)-ethylenediamine-N,N-diacetic acid, N,N'-bis(2-hydroxybenzyl)ethylenediamine-N,N'-diacetic acid ketone, monobenzylamino diacetic acid, N,N'-bis(3-aminopropyl)ethylenediamine, desferal, N'-{5-[acetyl(hydroxyl)amino]pentyl}-N- [5-({4-[5-aminopentyl)(hydroxyl)amino]-4-oxobutanoyl}amino)pentyl]-N-hydroxylsuccinamide, 1,4,7-triazacyclononane, 1,4,7-triazacyclononane-1,7-diacetic acid/1,4,7-triazacyclononane- 1,4,7-triacetic acid maleimide, 1,4,7,10-tetraazacyclododecane-1,7-diacetic acid, 1,4,7,10-tetraazacyclododecane, sodium 2,2',2"-(1,4,7,10-tetraazacyclododecane-1,4,7- triyl)triacetate, 1,4,7,10-tetraazacyclododecane-1,4,7-triacetic acid-10-methylphosphonic acid, 6-hydrazinonicotinic acid N-hydroxysuccinimide ester hydrochloride, S-acetylmercaptoacetyltriserine, mercaptoacetyltriglycine, isonitrile, and derivatives thereof.

4. The PSMA-targeting ligand compound or the stereoisomer, the tautomer, the hydrate, the solvate, the pharmaceutically acceptable salt or ester thereof as claimed in claim 1, wherein R₁ is selected from the group consisting of the following groups: 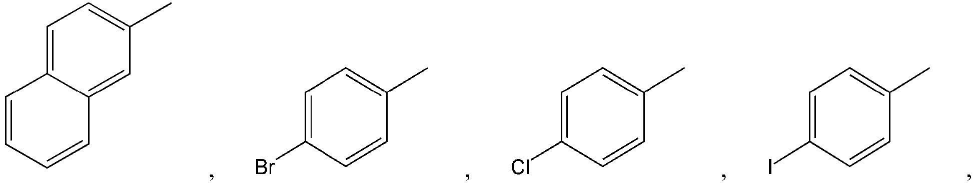 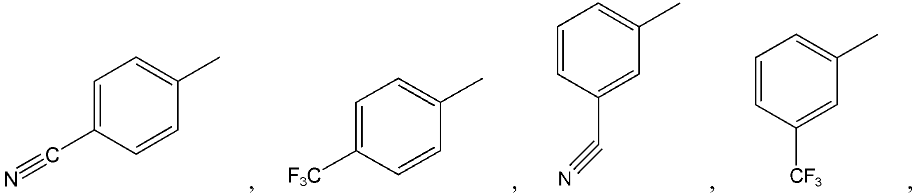 and 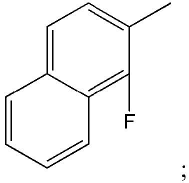 and
Y is

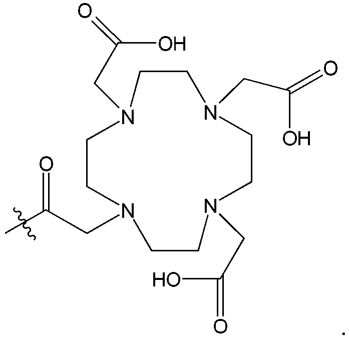

5. The PSMA-targeting ligand compound or the stereoisomer, the tautomer, the hydrate, the solvate, the pharmaceutically acceptable salt or ester thereof as claimed in any one of claims 1-4, wherein the PSMA-targeting ligand compound or the stereoisomer thereof is selected from the group consisting of the following compounds: 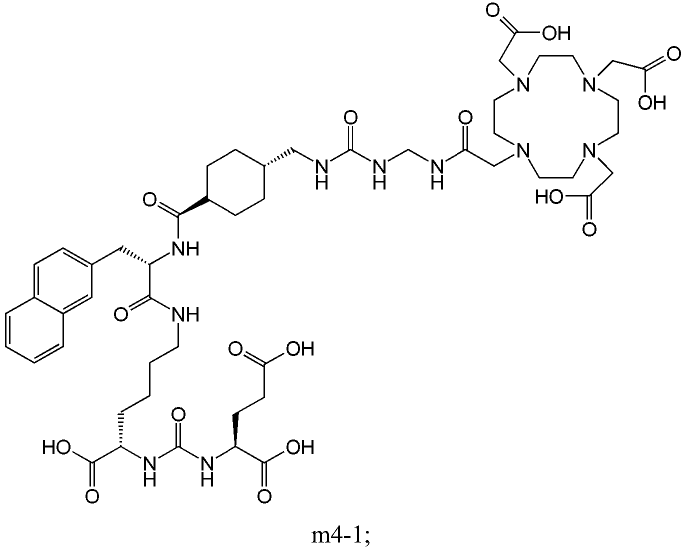 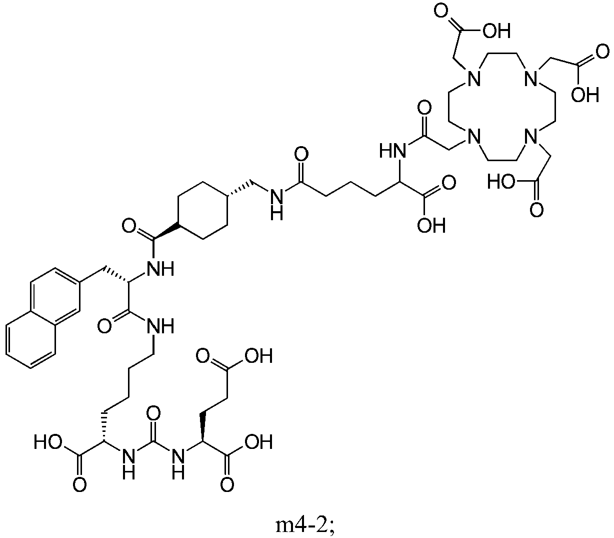 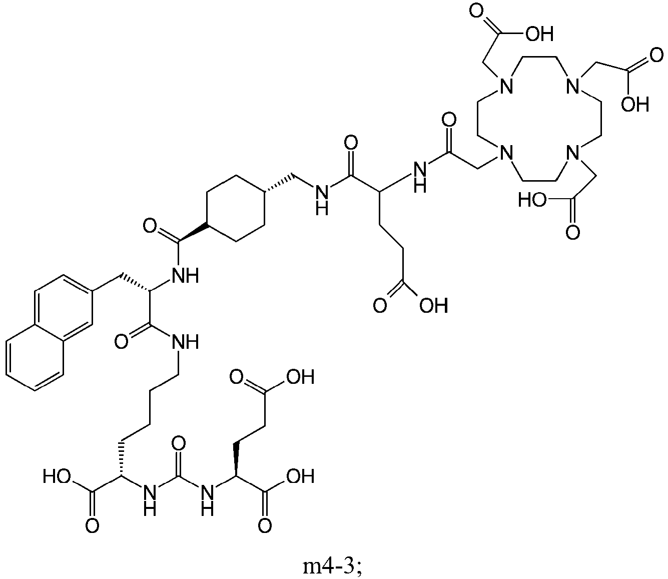 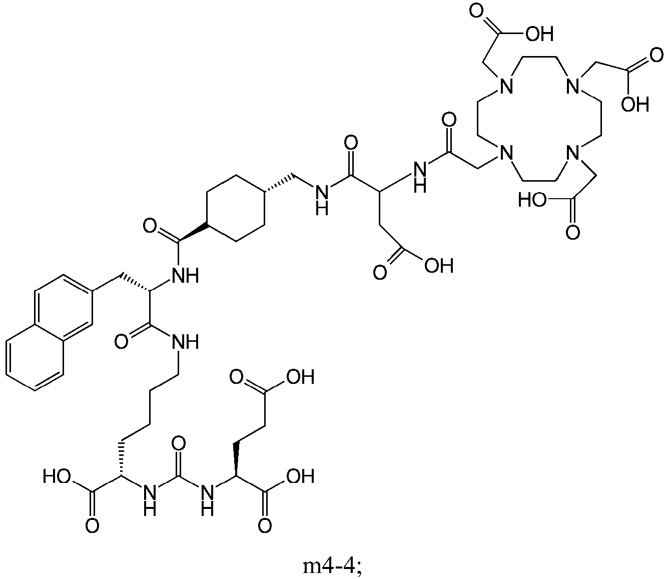 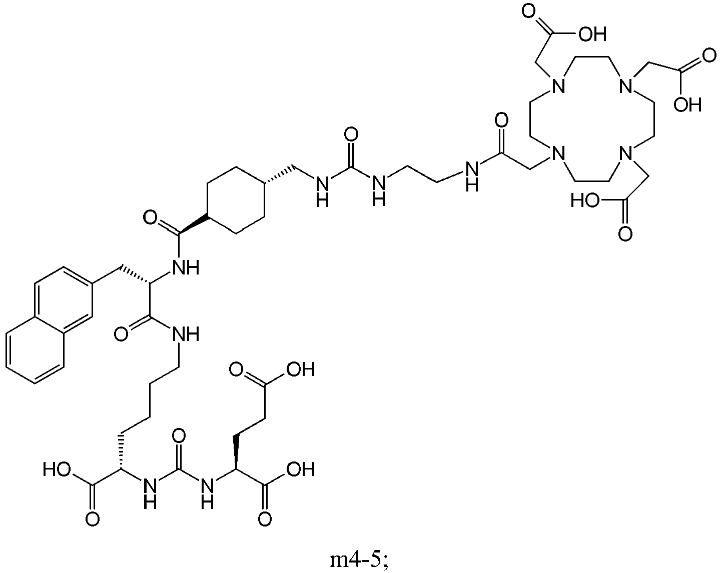 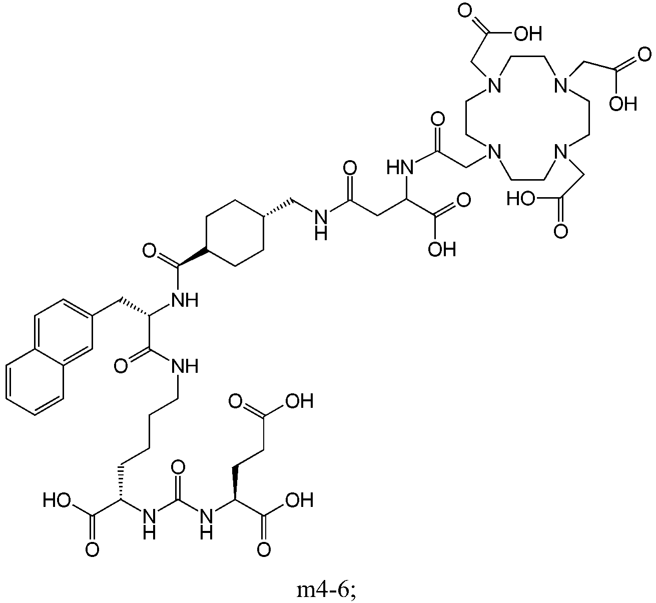 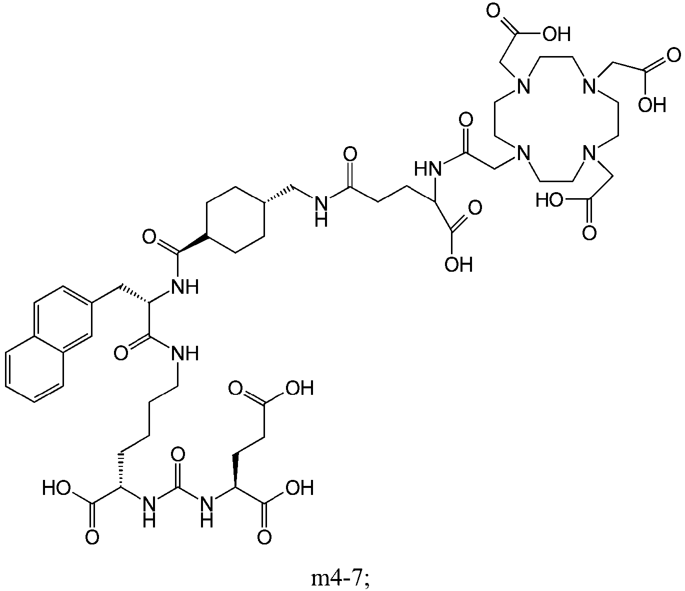 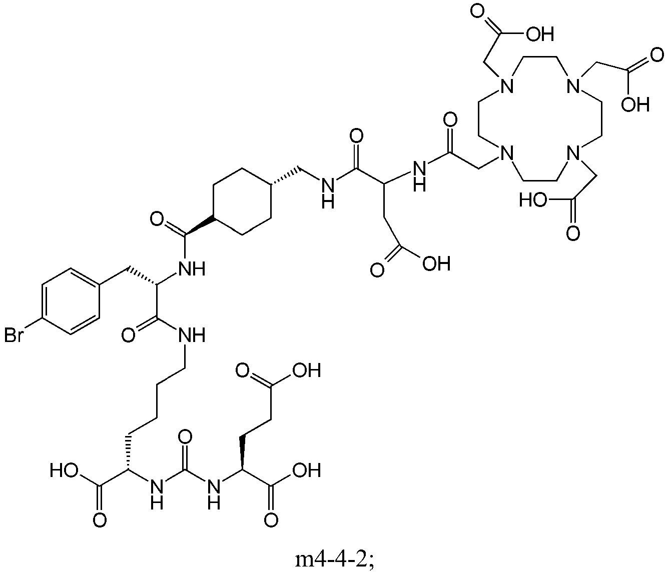 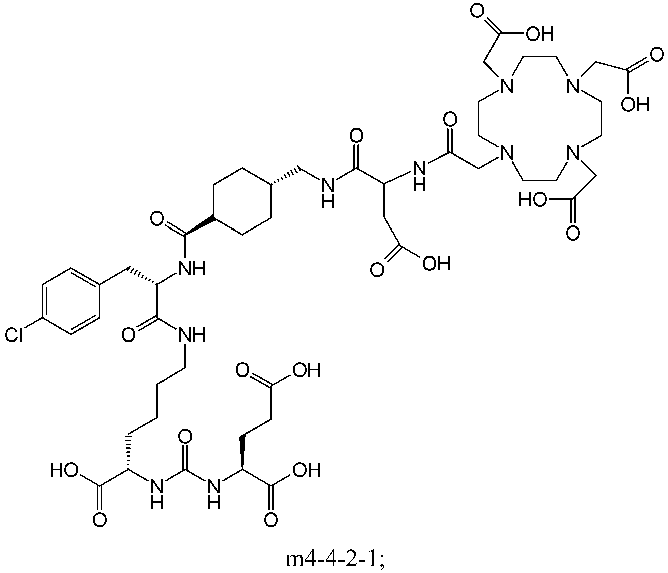 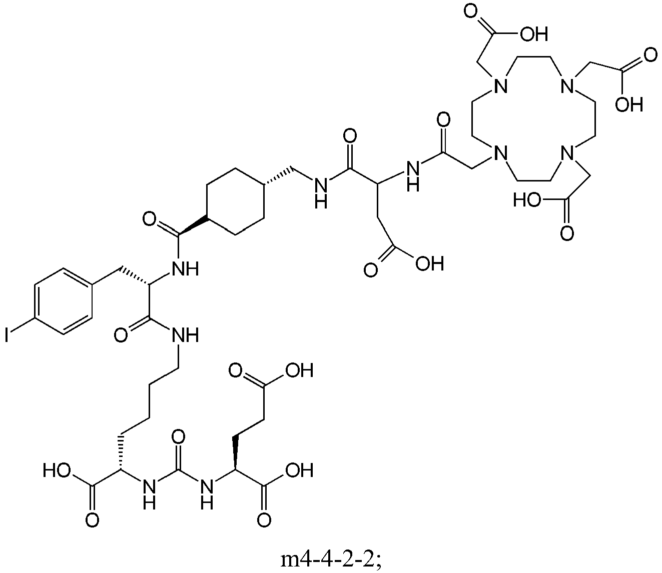 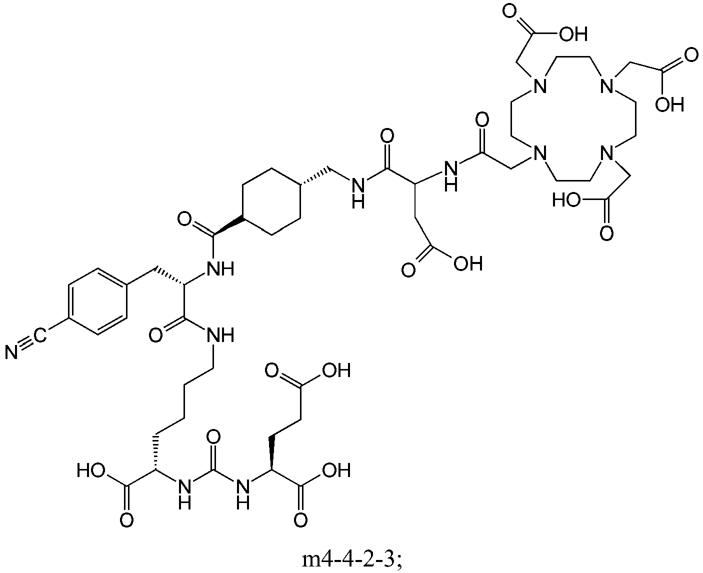 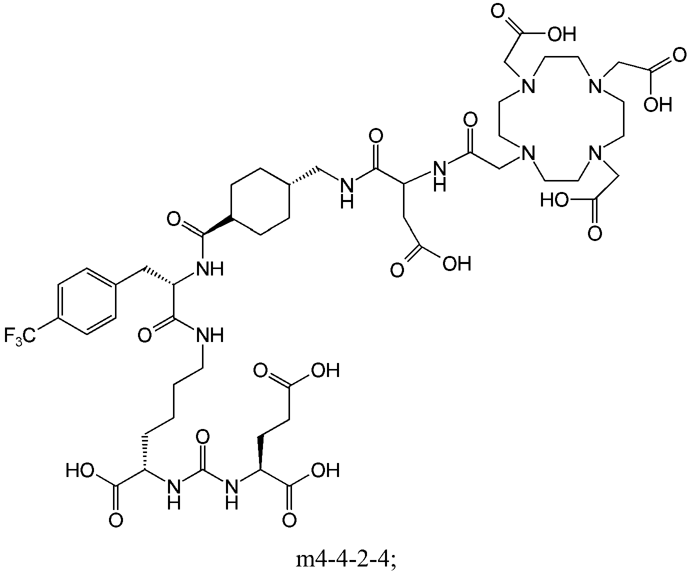 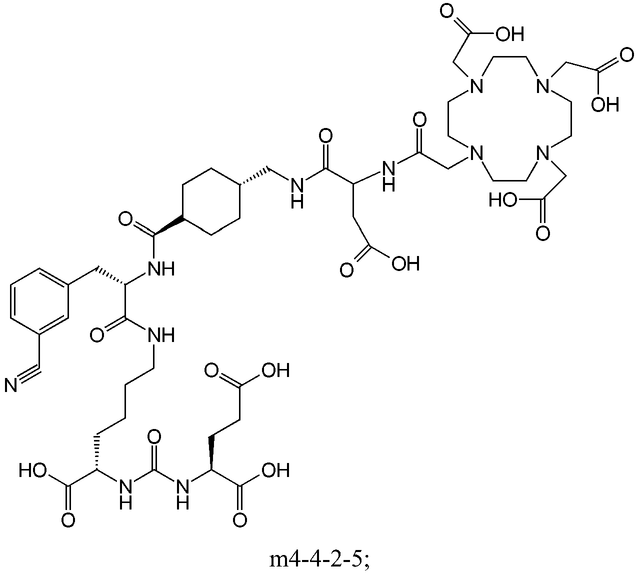 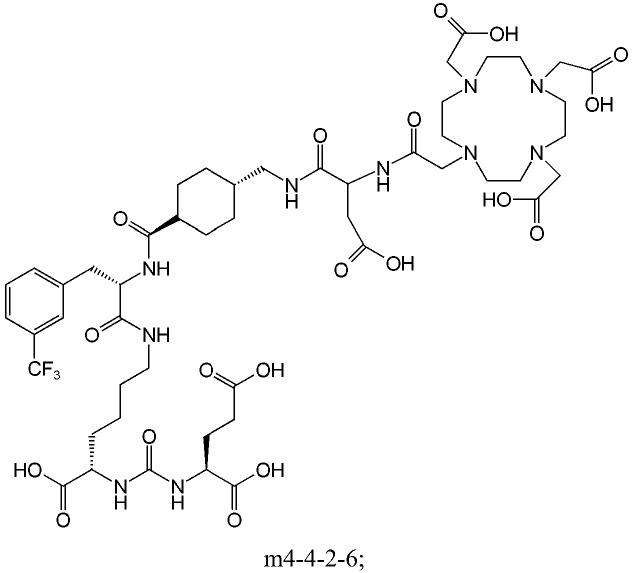 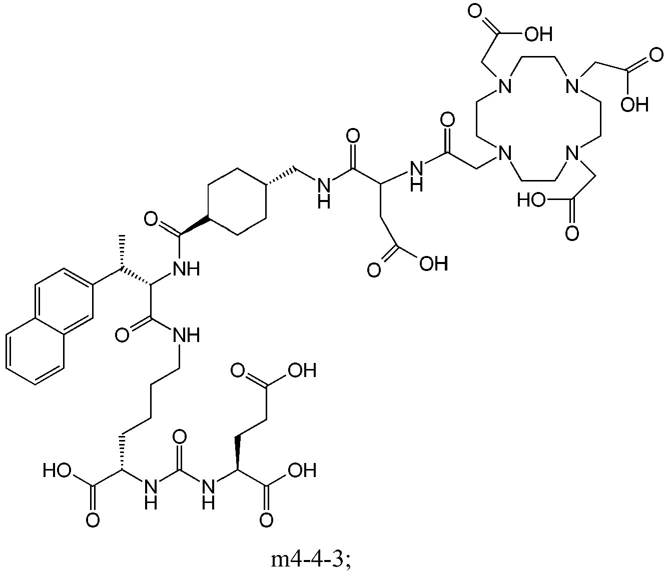 and

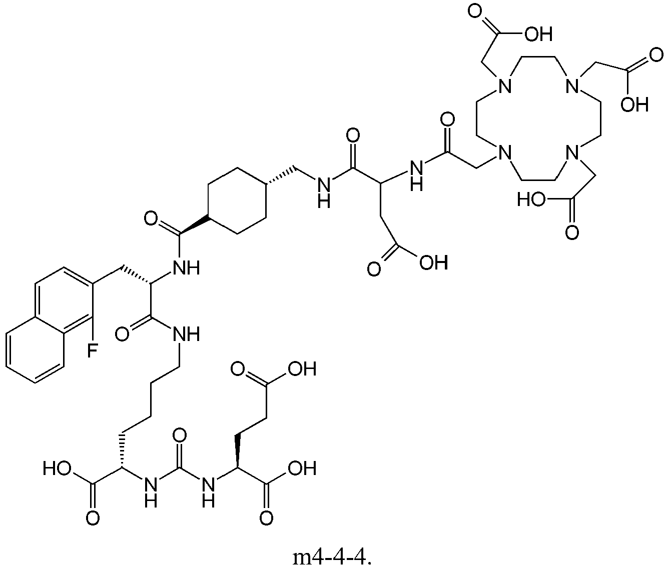

6. A method for preparing the PSMA-targeting ligand compound or the stereoisomer, the tautomer, the hydrate, the solvate, the pharmaceutically acceptable salt or ester thereof as claimed in any one of claims 1-5, the method comprising the steps of:
(1) reacting a compound as shown in formula II with a compound as shown in formula III in an organic solvent containing a condensing reagent and an organic amine to produce a compound as shown in formula IV, 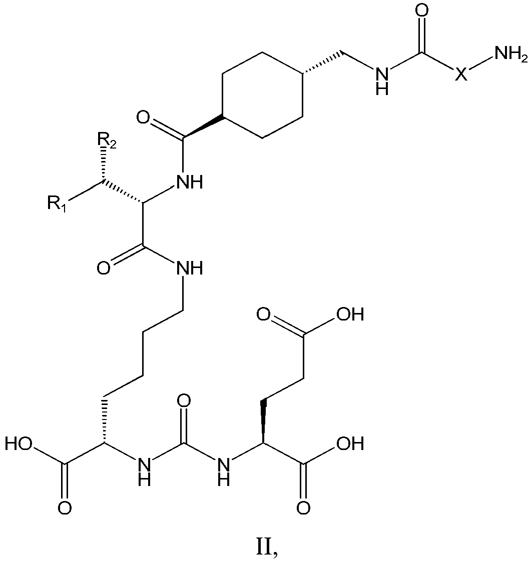 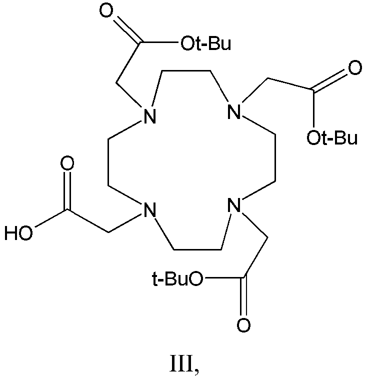 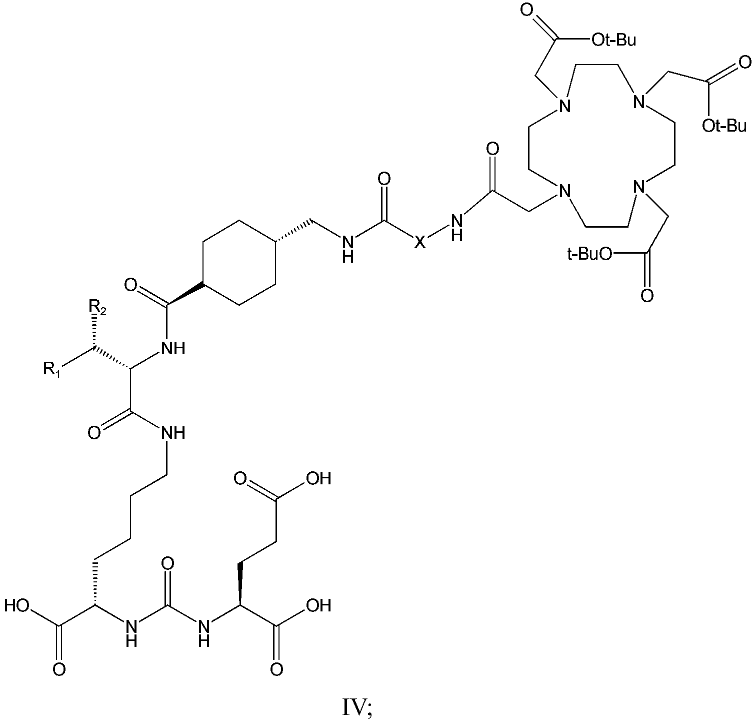 and
(2) reacting the compound as shown in formula IV in an organic solvent containing a deprotection reagent to produce the PSMA-targeting ligand compound as shown in formula I.

7. The method as claimed in claim 6, wherein the condensing reagent is at least one selected from the group consisting of 2-(7-azobenzotriazole)-N,N,N',N'-tetramethyluronium hexafluorophosphate, N,N'-dicyclohexylcarbodiimide, 1-(3-dimethylaminopropyl)-3-ethylcarbodiimide, (benzotriazol-1-yl-oxy)tripyrrolidnophosphonium hexafluorophosphate, and 1-propylphosphonic cyclic anhydride;
the organic amine is one of N,N-diisopropylethylamine and triethylamine, or a mixture of the N,N-diisopropylethylamine and the triethylamine;
the deprotection reagent is at least one selected from the group consisting of trifluoroacetic acid, a solution of hydrogen chloride in dioxane, a solution of hydrogen chloride in methanol, and a solution of hydrogen chloride in ethyl acetate; and
the organic solvent is at least one selected from the group consisting of N,N-dimethylformamide, tetrahydrofuran, and dichloromethane.

8. A chelate, comprising:
the PSMA-targeting ligand compound or the stereoisomer, the tautomer, the hydrate, the solvate, the pharmaceutically acceptable salt or ester thereof as claimed in any one of claims 1-5; and
a radioactive metal ion chelated thereto.

9. The chelate as claimed in claim 8, wherein a radioactive metal is one or more selected from the group consisting of ¹⁷⁷Lu, ¹²⁵I, ¹³¹I, ²¹¹At, ¹¹¹In, ¹⁵³Sm, ¹⁸⁶Re, ¹⁸⁸Re, ⁶⁷Cu, ²¹²Pb, ²²⁵Ac, ²¹³Bi, ²¹²Bi, ²¹²Pb, and ⁶⁸Ga;
preferably, the radioactive metal is ¹⁷⁷Lu.

10. A pharmaceutical composition for use in treatment of a tumor, the pharmaceutical composition comprising the chelate as claimed in claim 8 or 9, and optionally at least one pharmaceutically acceptable carrier.

11. A reagent composition for use in diagnosis of a tumor, the reagent composition comprising the chelate as claimed in claim 8 or 9, and optionally at least one diagnostically acceptable carrier.

12. A method for preparing a PSMA inhibitor, comprising:
using the PSMA-targeting ligand compound or the stereoisomer, the tautomer, the hydrate, the solvate, the pharmaceutically acceptable salt or ester thereof as claimed in any one of claims 1-5.

13. Chelate as claimed in claim 8 or 9 for use in treatment of a tumor.

14. A method for preparing a reagent for diagnosis of a tumor, comprising: using the chelate as claimed in claim 8 or 9.

15. The method as claimed in claim 14, wherein the reagent for the diagnosis of the tumor is a tumor imaging agent,
preferably, the tumor is at least one selected from the group consisting of prostate cancer, renal carcinoma, and gastric cancer;
preferably, the prostate cancer is at least one selected from the group consisting of castration-resistant prostate cancer, metastatic castration-resistant prostate cancer, and PSMA-positive prostate cancer.
